(19) Europäisches Patentamt / European Patent Office / Office européen des brevets

(11) **EP 4 428 554 B1**

(12) **EUROPÄISCHE PATENTSCHRIFT**

(45) Veröffentlichungstag und Bekanntmachung des Hinweises auf die Patenterteilung:
**11.03.2026   Patentblatt 2026/11**

(21) Anmeldenummer: **24152815.7**

(22) Anmeldetag: **19.01.2024**

(51) Internationale Patentklassifikation (IPC):
**G01R 31/62** (2020.01)    **G06F 17/18** (2006.01)
**G06F 119/04** (2020.01)

(52) Gemeinsame Patentklassifikation (CPC):
**G01R 31/62**; G06F 2119/04

(54) **VERFAHREN ZUR ALTERUNGSBEURTEILUNG UND INSBESONDERE ZUSTANDSÜBERWACHUNG, COMPUTERPROGRAMM UND COMPUTERLESBARES MEDIUM**

METHOD FOR ASSESSING AGING AND PARTICULARLY FOR MONITORING CONDITION, COMPUTER PROGRAM AND COMPUTER-READABLE MEDIUM

PROCÉDÉ D'ÉVALUATION DU VIEILLISSEMENT ET NOTAMMENT DE SURVEILLANCE D'ÉTAT, PROGRAMME INFORMATIQUE ET SUPPORT LISIBLE PAR ORDINATEUR

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **08.03.2023   DE 102023202056**

(43) Veröffentlichungstag der Anmeldung:
**11.09.2024   Patentblatt 2024/37**

(73) Patentinhaber: **Siemens Energy Global GmbH & Co. KG**
**81739 München (DE)**

(72) Erfinder:
• **Raith, Johannes**
  **8162 Passail (AT)**
• **Scala, Mario**
  **8010 Graz (AT)**

(56) Entgegenhaltungen:
**CN-A- 112 257 232**

• **JOHANNES RAITH ET AL: "Simulation of long-term transformer operation with a dynamic thermal, moisture and aging mode I", 5TH INTERNATIONAL COLLOQUIUM ON TRANSFORMER RESEARCH AND ASSET MANAGEMENT; INTERNATIONAL COLLOQUIUM TRANSFORMER RESEARCH AND ASSET MANAGEMENT; OPATIJA, CROATIA; OCTOBER 9-12, 2019, SINGAPORE, 17 July 2020 (2020-07-17), pages 211 - 226, XP009554325, ISBN: 978-981-15-5599-2, DOI: 10.1007/ 978-981-15-5600-5 17**
• **YAMAGATA N ET AL: "Diagnosis of thermal degradation for thermally upgraded paper in mineral oil", CONDITION MONITORING AND DIAGNOSIS, 2008. CMD 2008. INTERNATIONAL CONFERENCE ON, IEEE, PISCATAWAY, NJ, USA, 21 April 2008 (2008-04-21), pages 1000 - 1004, XP031292665, ISBN: 978-1-4244-1621-9**

# EP 4 428 554 B1

**Beschreibung**

Die Erfindung betrifft ein Verfahren zur Alterungsbeurteilung und insbesondere Zustandsüberwachung eines eine feste Isolationsanordnung und ein mit der festen Isolationsanordnung in Kontakt stehendes flüssiges und/oder gasförmiges Isoliermedium umfassenden elektrischen Gerätes, bevorzugt eines Transformators oder einer Drossel. Darüber hinaus betrifft die Erfindung ein Computerprogramm sowie ein computerlesbares Medium.

Isolationssysteme elektrischer Geräte bestehen oft aus einer Kombination aus einem Isoliermedium, das flüssig bzw. gasförmig ausgebildet sein kann, und einer festen Isolationsanordnung, letztere typischerweise auf Zellulosebasis. Substanzen, die aufgrund der Alterung der festen Isolationsanordnung entstehen, in das Isoliermedium übergehen und dort detektiert werden können, werden auch als "Aging-Marker" betrachtet. Allerdings fehlt der quantitative Rückschluss auf die Belastung der am stärksten beanspruchten Bereiche, da die messtechnische detektierte Information nur einen integralen Wert liefert. Somit beschränkt sich eine Aussagefähigkeit z.B. auf eine Ampelfunktion mit Grenzwerten. Insbesondere fehlt eine zuverlässige Zuordnung, ob eine messtechnisch beobachtete Menge an "Aging-Markern" im Einklang mit dem Betrieb steht, oder ob ein Verdacht auf ein fehlerhaftes Verhalten des elektrischen Gerätes vorliegt.

Der Anmelderin ist bekannt, dass teilweise versucht wird, eine Anpassung integraler Messergebnisse durch empirische Faktoren vorzunehmen, um so auf einen Alterungszustand im wärmsten bzw. kritischsten Bereich (sogenannter "Hotspot") schließen zu können. Dieser Ansatz kann jedoch allenfalls sehr grobe Aussagen liefern, da die Temperaturverteilung verschiedenen Parametern unterliegt, die vom Design des elektrischen Gerätes, z.B. Transformators abhängt, und die sich während des Betriebes ändern können.

CN 112 257 232 A offenbart ein Verfahren zur Erstellung eines Lebensdauermodells auf der Grundlage des Ethanolgehalts in Öl und des Polymerisationsgrads von Transformatorenpapier. Ethanol ist als charakteristischen Index für die Lebensdauerbewertung des Transformatorisolierpapiers angenommen. Eine theoretische Grundlage für die Darstellung des Überhitzungsfehlers des Transformators und die Beurteilung des Isolationszustands am heißen Punkt der Wicklung aufgrund der besonderen Empfindlichkeit des Ethanols im Öl gegenüber hohen Temperaturen ist präsentiert.

Aus dem Artikel "Simulation of long-term transformer operation with a dynamic thermal, moisture and aging model", 5th International Colloquium on Transformer Research and Asset Management, October 2018, Opatija, Croatia (weitere Veröffentlichung des Artikels: 5th International Colloquium on Transformer Research and Asset Management, Lecture Notes in Electrical Engineering, vol 671. Springer, Singapore, 2020, https://doi.org/10.1007/ 978-981-15-5600-5_17, geht ein thermo-hydraulisches Alterungsmodell (englisch: Thermo-Hydraulic Aging Model, THAM) für einen Transformator hervor. Mit diesem wird in einer Fallstudie der Langezeitbetrieb eines Transformators simuliert, von dem sich u.a. Wicklungen, der Kern und eine Kühlanordnung in einem mit Öl als flüssigem Isoliermedium befüllten Tank befinden. Kern und Wicklungen sowie weitere Transformatorkomponenten sind hier mit einer Zellulose-basierten festen Isolationsanordnung versehen. Diese umfasst Pressboard und Papier, mit dem Leiter umwickelt sind. Die Komponenten werden mit verschiedenen Zweigen mit spezifischen Eigenschaften, die durch Knoten verbunden sind, modelliert, um ein thermo-hydraulisches Netzwerkmodell zu erhalten. Es werden an unterschiedlichen Stellen bzw. Positionen der festen Isolationsanordnung Temperaturen und sich daraus ergebende lokale Feuchtewerte gerechnet, aus der durch Anwendung einer Alterungsformel sich lokale DP-Zahlen ergeben.

Aus der WO 99/60682 A1 gehen ein Verfahren und eine Anordnung zur Ermittlung von Zustandsgrößen hervor. Für die Ermittlung von Temperaturen in einem ölgekühlten Transformator werden die Transformatorklemmenspannungen, die Wicklungsströme und die Umgebungstemperatur gemessen und der Status von Lüftern und Pumpen und die Schalterstellung eines Stufenschalters festgestellt. Die gemessenen und festgestellten Größen werden einem thermohydraulischen Modell zugeführt, in dem mit Hilfsgrössen, welche Verluste im Transformator, Wärmeübergangsparameter, Strömungswiderstände und die Ölströmung sind, und einem hydraulischen Netzwerk des Ölkreislaufes, welches Zweige und Knoten aufweist, Zustandsgrößen berechnet werden. Die Zustandsgrößen sind die mittleren Temperaturen und die Hotspot-Temperaturen in verlusterzeugenden Transformatorteilen und die mittleren Öltemperaturen in Zweigen und in Knoten des hydraulischen Netzwerkes des Ölkreislaufes.

Es ist eine Aufgabe der vorliegenden Erfindung, ein Verfahren der eingangs genannten Art anzugeben, welches eine zuverlässigere Alterungsbeurteilung und insbesondere Unterscheidung zwischen einem normalen und einem fehlerbehafteten Betrieb eines elektrischen Gerätes mit einem Isolationssystem aus fester Isolationsanordnung sowie flüssigem bzw. gasförmigem Isoliermedium und somit einen besonders sicheren Betrieb ermöglicht.

Diese Aufgabe wird gelöst durch ein Verfahren zur Alterungsbeurteilung und insbesondere Zustandsüberwachung eines eine feste Isolationsanordnung und ein mit der festen Isolationsanordnung in Kontakt stehendes flüssiges und/oder gasförmiges Isoliermedium umfassenden elektrischen Gerätes, bevorzugt eines Transformators oder einer Drossel, bei dem

> S1) ein thermo-hydraulisches Alterungsmodell des elektrischen Gerätes bereitgestellt und/oder erstellt und mit dem Alterungsmodell eine Simulation für das elektrische Gerät durchgeführt wird,

S2) im Rahmen der Simulation für verschiedene Bereiche des elektrischen Gerätes, insbesondere für verschiedene Bereiche der festen Isolationsanordnung und/oder für verschiedene Bereiche des von dem Isoliermedium eingenommenen Volumens, lokale Temperaturen berechnet werden, bevorzugt, wobei unter Berücksichtigung der lokalen Temperaturen und optional weiterer alterungsbestimmender Einflussgrößen, insbesondere der lokalen Feuchte und/oder des Sauerstoffgehaltes des Isoliermediums, für verschiedene Bereiche der festen Isolationsanordnung lokale Alterungsgrößen, insbesondere lokale DP-Zahlen, berechnet werden,
S3) für die verschiedenen Bereiche der festen Isolationsanordnung unter Berücksichtigung der berechneten lokalen Temperaturen und/oder, sofern in Schritt S2 berechnet, der lokalen Alterungsgrößen und bevorzugt unter zusätzlicher Berücksichtigung von Massen der festen Isolationsanordnung, Mengen von wenigstens einem Alterungsprodukt, das aufgrund der Alterung der festen Isolationsanordnung entsteht und in das Isoliermedium übergeht, berechnet werden, bevorzugt, wobei die berechneten Alterungsproduktmengen herangezogen werden, um zwischen einem normalen und einem fehlerhaften Zustand des elektrischen Gerätes zu unterscheiden.

Die berechneten Alterungsproduktmengen werden zweckmäßigerweise herangezogen, um zwischen einem normalen und einem fehlerhaften Zustand des elektrischen Gerätes zu unterscheiden.

Als besonders zweckmäßig hat sich erwiesen, wenn Simulationsergebnisse einem Vergleich mit messtechnisch erfassten Werten unterzogen werden. So ist in vorteilhafter Ausgestaltung vorgesehen, dass ein Vergleich der in Schritt S3 berechneten Alterungsproduktmengen, insbesondere einer Summe von berechneten Alterungsproduktmengen, mit einer oder mehreren an dem elektrischen Gerät messtechnisch erfassten Alterungsproduktmengen erfolgt und anhand des Vergleichsergebnisses zwischen einem normalen und einem fehlerhaften Zustand des elektrischen Gerätes unterschieden wird.

Kerngedanke der vorliegenden Erfindung ist es mit anderen Worten, die (ortsabhängige) Erzeugung und die Verteilung eines oder mehrerer Alterungsprodukte, die bei einem Isolationssystem aus fester Isolation, z.B. Papier, und einem flüssigen und/oder gasförmigen Isoliermedium, z.B. Öl, im Laufe des Betriebes entstehen und insbesondere von der festen Isolation in das Isoliermedium übergehen und/oder im Isoliermedium selber entstehen ("Aging-Marker") mittels eines Simulationsmodells zu berechnen. Zweckmäßigerweise wird dann anhand des/eines Simulationsergebnisses, bzw. eines Teils eines solchen, darauf geschlossen, ob ein fehlerhafter oder normaler Betrieb des elektrischen Gerätes vorliegt. Das bzw. die Alterungsprodukte können an einem realen elektrischen Gerät auf vergleichsweise einfache Weise messtechnisch erfasst werden, etwa durch Entnahme einer Probe des Isoliermediums, z.B. Öls. Damit ist ein Vergleich zwischen Simulations- und Messwerten möglich und es können aussagekräftige, zuverlässigere Rückschlüsse auf den Zustand des elektrischen Gerätes gezogen werden.

Zweckmäßigerweise erfolgt eine Simulation von inneren Vorgängen in dem elektrischen Gerät. Es kann auch ein Betrieb des elektrischen Gerätes simuliert werden.

Im Rahmen der Erfindung werden dabei für verschiedene Bereiche bzw. Stellen des elektrischen Gerätes, insbesondere für verschiedene Teile dieses, lokale Temperaturen berechnet. Es erfolgt bevorzugt eine Netzwerkberechnung der Temperaturen in einem elektrischen Gerät, wie etwa ein Transformator oder einer Drossel. Die lokalen Temperaturen werden zweckmäßigerweise bei der optionalen Ermittlung der lokalen Alterungsgrößen berücksichtigt, fließen beispielsweise in Gleichungen ein, die für die Berechnung von Alterungsgrößen genutzt werden.

In besonders zweckmäßiger Ausgestaltung des erfindungsgemäßen Verfahrens ist das thermo-hydraulische Alterungsmodell entsprechend als Netzwerkmodell ausgebildet, insbesondere als Netzwerkmodell für eine Netzwerkberechnung lokaler Temperaturen an verschiedenen Stellen/Bereichen eines elektrischen Gerätes, etwa Transformators.

Weiter bevorzugt gilt, dass das Netzwerkmodell ausgebildet ist, um mit bzw. in diesem die Umverteilung der lokalen Alterungsprodukt-Erzeugung, etwa $CO_2$-Beiträgen, auf alle Bereiche bzw. Stellen oder Teile der festen Isolationsanordnung zu berechnen, um im Gesamtsystem ins Gleichgewicht zu kommen.

Beispielsweise muss nicht die lokale Isolation mit der höchsten $CO_2$-Erzeugungsrate automatisch auch den höchsten spezifischen $CO_2$-Gehalt haben. Mit anderen Worten: durch Nutzung eines Netzwerkmodells werden diese Ausgleichsvorgänge berechenbar, indem das strömende Medium, z.B. Isolieröl, als Transportmedium für z.B. $CO_2$ die lokalen Positionen der festen Isolationsanordnung miteinander verbindet. Dabei erfolgt ein Austausch von z.B. $CO_2$, um ein Gleichgewicht zwischen lokaler Isolationsanordnung und umgebendem Isoliermedium zu erzielen. Dieser Austausch kann in beide Richtungen stattfinden - z.B. vom Öl in die Zellulose und umgekehrt - da das Speichervermögen von dem bzw. den Alterungsprodukten, etwa von $CO_2$, in der festen Isolationsanordnung, etwa Zellulose, in der Regel nicht vernachlässigbar klein ist. Aus den lokalen Werten des wenigstens einen im Isoliermedium gelösten Alterungsproduktes, z.B. in Öl gelöstem $CO_2$, kann ein Alterungsprodukt-Gehalt im Gesamt-Isoliermedium erhalten werden, ein Wert, der messtechnisch zugänglich ist. Das Netzwerkmodell ist zweckmäßigerweise entsprechend ausgestaltet.

Anstelle einer empirischen Bewertung erfolgt eine systematischere Beurteilung eines Alterungszustandes und insbesondere eine Überwachung des Alterungszustandes. Die Leistungsfähigkeit von Simulationsmodellen wird ausgebaut, indem anstelle integraler Aussagen eine Bewertung einzelner Teile stattfindet. Die Anwendung eines thermo-hydraulischen Temperaturmodells wird erweitert. Insbesondere durch die Alterungssimulation über die Berechnung von lokaler DP-Zahl, die im Allgemeinen nicht mit einer direkten Messung überprüft werden kann, ergibt sich durch die erfindungsgemäße Berechnung der Alterungsprodukte ein Link zu den Messwerten dieser Alterungsprodukte im Isoliermedium, etwa Öl.

Eine Zuordnung, ob eine messtechnisch beobachtete Menge im Einklang mit dem Betrieb steht oder ein Verdacht auf ein fehlerhaftes Verhalten im Gerät besteht, wird möglich. Durch die erfindungsgemäße erfolgende Simulation finden dabei die Alterungsprozesse beeinflussende Größen und deren räumliche Charakteristik, insbesondere die Temperaturverteilung, Feuchteverteilung und daraus abgeleitete lokale Erzeugung von Alterungsprodukten sowie optional lokale DP-Zahl-Verteilung, insbesondere mit zugehörigen Isolationsmassen, Berücksichtigung.

In bevorzugter Ausgestaltung werden in Schritt S3 Mengen von 2-FAL und/oder $CO_2+CO$ als Alterungsproduktmengen mittels des thermo-hydraulischen Alterungsmodells berechnet.

Diese Größen stellen besonders geeignete "Aging-Marker" dar, die im Rahmen des erfindungsgemäßen Verfahrens betrachtet werden können. Auf der anderen Seite lassen sich diese Größen in einem Isoliermedium auch messtechnisch gut erfassen. In der Regel wird 2-FAL hauptsächlich dann sichtbar, wenn bereits eine deutliche Alterung vorliegt, während $CO_2+CO$ schon bei Beginn entsteht.

Bei der Berechnung der Alterungsproduktmengen für die verschiedenen Stellen bzw. Bereiche der festen Isolationsanordnung, insbesondere verschiedene Teile dieser, werden zweckmäßigerweise die Massen der verschiedenen Bereiche bzw. Teile der Isolationsanordnung berücksichtigt. Dies, da die von der festen Isolationsanordnung bzw. Teilen dieser in das Isoliermedium übergehenden Alterungsprodukte in der Regel von der (jeweiligen) Masse abhängen werden.

Das Isoliermedium hat zweckmäßigerweise sowohl isolierende als auch kühlende Eigenschaften, dient mit anderen Worten insbesondere sowohl zur Isolierung als auch Kühlung.

Das erfindungsgemäß zum Einsatz kommende thermo-hydraulische Alterungsmodell ist bevorzugt mehrstufig ausgebildet bzw. kann mehrere Untermodelle umfassen. Es beinhaltet insbesondere:

- die Berechnung der elektrischen Wirkverluste in den verschiedenen Teilen des elektrischen Gerätes (z.B. Transformator, Drossel, etc.) auf Grund der anliegenden Spannung und des Stroms,

  - die Berechnung von dynamischen lokalen Temperaturen in unterschiedlichen Teilen des Isolationssystems umfassend die festen Isolationsanordnung und das flüssige oder gasförmige Isoliermedium,
  - (optional) die Berechnung der Alterung der festen Isolationsanordnung, sofern Zellulose-basiert insbesondere der Zellulosealterung, die typischerweise über die DP-Zahl ausgedrückt wird, an unterschiedlichen Stellen/Teilen der festen Isolationsanordnung auf Grund deren lokalen Temperaturen und bevorzugt (weiterer) alterungsbestimmenden Einflussgrößen, wie Sauerstoff und lokale Feuchte,
  - die Berechnung der erzeugten Alterungsprodukte infolge der Isolationsalterung insbesondere durch Abnahme der DP-Zahlen an unterschiedlichen Teilen der festen Isolation unter Berücksichtigung deren Massen.

Die Abkürzung "DP" bei der DP-Zahl steht in hinlänglich bekannter Weise für "Degree of Polymerization". Es ist insbesondere ein Maß der abnehmenden Länge von Zellulosemolekülen durch thermische Alterung, wodurch die mechanische Festigkeit beeinträchtigt wird, was zu einem Betriebsrisiko führen kann. Die DP-Zahl gibt eine Anzahl von Basis-Moleküleinheiten in einer Zellulosekette an, die sich durch Polymerisation, der Aneinanderreihung des Basismoleküls, bildet. Neue Zellulose hat DP-Werte von über 1000, während durch chemische Zersetzung die Ketten in kürzere Einheiten zerbrechen, was deren mechanische Festigkeit reduziert. Bei DP-Werten unter 200 wird die mechanische Festigkeit, wie z.B. die Zugfestigkeit, deutlich verschlechtert, sie liegt deutlich unter 50% gegenüber dem Anfangszustand, was den sicheren Betrieb eines Trafos gefährden könnte, insbesondere bei Kurzschlüssen im Netz, die zumindest kurzzeitig zu großen Strömen und damit beanspruchenden Kräften bzw. Schwingungen führen.

Für die optionale Berechnung von DP-Zahlen als Alterungsgrößen, insbesondere die Berechnung der durch Alterung abnehmenden DP-Zahl, wird bevorzugt wenigstens eine Formel genutzt, welche als Einflussgrößen den Anfangszustand $DP_{Start}$, die Zeitdauer t, die Temperatur $\Theta h$ der festen Isolation, insbesondere Zellulose, im Hotspot und eine Materialeigenschaft A der Zellulose, die von $H_2O$ und O sowie der Zellulosequalität abhängt, umfasst. Bei Papieren gibt es als zweite Zellulosequalität eine thermostabilisierte Qualität mit reduzierter thermischer Alterung bei hohen Temperaturen. Diesbezüglich wird auch auf die Norm IEC 60076-7:2018 "Loading guide for mineral-oil-immersed power transformers", 2018, verwiesen, und darin insbesondere auf die Seiten 42 mit der dortigen Gleichung A.1 sowie Tabelle A.1 auf Seite 43, deren Parameter auf einfache Weise die Temperatur, Feuchtigkeit, Zellulosequalität und Sauerstoffeinfluss berücksichtigen. Das erfindungsgemäß zum Einsatz kommende thermo-hydraulisches Alterungsmodell kann wenigstens eine solche Gleichung umfassen.

Alternativ oder zusätzlich kann vorgesehen sein, dass für die optionale Berechnung lokaler DP-Zahlen in Schritt S2 folgende Gleichung genutzt wird:

$$DP_t = \left[DP_{t-1}^{1-p(T)} + O_t \cdot M_t \cdot \Delta t \cdot (p(T) - 1)\right]^{\frac{1}{1-p(T)}}$$

Darin ist $M_t$ ein Feuchtigkeitsfaktor, p(T) eine Temperaturfunktion und Ot ein Sauerstoff-Faktor, welche den DP-abfall gemäß der Gleichung stark beeinflussen.

Für die Temperaturfunktion gilt bevorzugt:

| Nicht thermostabilisiertes Papier und Zellulose | Thermostabilisiertes Papier |
|---|---|
| $p(T) = 1{,}182455 \cdot e^{0{,}005791 \cdot T}$ | $p(T) = 1{,}024508 \cdot e^{0{,}006021 \cdot T}$ |

Für den Sauerstoff-Faktor wird in weiterer vorteilhafter Ausgestaltung folgende Gleichung genutzt:

$$O(T, ppm) = \quad 1 \; + \; (O_T - 1) \cdot O_{ppm}/O_{sat} \quad ,$$

mit $O_{sat}$=37000 ppm (< 1.000 m Meeresniveau, in Mineralöl) und $O_{ppm}$ gleich der im Transformator vorhandenen Sauerstoffkonzentration.

Ohne Messung kann insbesondere von zwei Möglichkeiten ausgegangen werden:

- offenes System mit Sauerstoff, der aus Luft nachgeliefert wird,
- geschlossenes System mit wenig Sauerstoff.

Bevorzugt gilt, dass das elektrische Gerät geschlossen ausgebildet ist, insbesondere durch ein System bzw. Gerät mit Luftabschluss gegeben ist.

Das thermo-hydraulische Alterungsmodell ist in zweckmäßiger Ausgestaltung dazu ausgebildet, sowohl das steady-state als auch das transiente Verhalten folgender Größen, insbesondere basierend auf der Last, zu berechnen:

- Den Wärmefluss und die Temperatur von Komponenten bzw. Bereichen des elektrischen Gerätes (im Falle eines Transformators insbesondere des Isoliermediums, etwa Öls, im oberen und unteren Bereich des Tanks bzw. der Kühler, Kerntemperaturen, Temperaturen von Wicklungsteilen sowie den lokalen Hot Spots (Heißpunktstellen) und lokale Öltemperaturen in den Wicklungen).
- Den Ölfluss im elektrischen Gerät bzw. in Komponenten dieses (im Falle eines Transformators im Kern, den Wicklungen, im Tank etc.), der durch den hydraulischen Widerstand, die Auftriebskraft und optional den Pumpendruck bestimmt ist.

Weiter bevorzugt ist das Modell ausgebildet, folgende Größen in Bezug auf die Feuchte und das Alterungsverhalten zu berechnen:

- Den Feuchtigkeitsaustausch zwischen fester Isolation und flüssigem bzw. gasförmigem Isoliermedium, bevorzugt einschließlich eines möglichen Feuchtigkeitsaustauschs mit der Atmosphäre durch Diffusion von Feuchtigkeit nach außen, bei einem Transformator insbesondere durch das Öldehngefäß (englisch: conservator).
- Berechnung der lokalen Alterungsgröße, insbesondere Polymerisationsgrades (DP-Wert) der verschiedenen Bereiche bzw. Zelluloseelemente unter Berücksichtigung von:

  ◦ dem Einfluss von Feuchtigkeit und Sauerstoff
  ◦ der Qualität der festen Isolation, insbesondere der Zellulosequalität (z.B. thermisch nicht stabilisiertes oder thermostabilisiertes (englisch: thermally upgraded) Papier)
  ◦ der Feuchtigkeitsbildung durch die Alterung selbst

- Einfluss der Alterung (DP-Wert) auf die Feuchtigkeitsaufnahme der festen Isolation, insbesondere Zellulose, und
- Risiko von Blasenbildung, wenn der Druck von gelösten Gasen und Feuchtigkeit in der festen Isolation oder dem Isoliermedium den darauf lastenden Umgebungsdruck übersteigt. Das kann zur Zerstörung des Gerätes führen.

Im Rahmen der vorliegenden Erfindung kann insbesondere eine erweiterte Version desjenigen thermo-hydraulischen Alterungsmodells zum Einsatz kommen, welches in dem Artikel "Simulation of long-term transformer operation with a dynamic thermal, moisture and aging model", 5th International Colloquium on Transformer Research and Asset Management, October 2018, Opatija, Croatia (weitere Veröffentlichung des Artikels: 5th International Colloquium on Transformer Research and Asset Management, Lecture Notes in Electrical Engineering, vol 671, Springer, Singapore, 2020, https://doi.org/10.1007/978-981-15-5600-5_17), offenbart ist. Dieses Simulationsmodell kann dabei derart erweitert sein bzw. werden, dass es zusätzlich eine Berechnung der Alterungsproduktmengen gemäß Schritt S3 ermöglicht bzw. umfasst, die aufgrund der Alterung der festen Isolationsanordnung entstehen (2-FAL und/oder $CO_2$+CO ) und in das Isoliermedium übergehen und über die Ausbreitung durch das Isoliermedium einen Gleichgewichtszustand im Gesamtsystem einnehmen, woraus ein im gesamten Isoliermedium einheitlicher Wert des im Isoliermedium gelösten Alterungsprodukts, dem Aging Marker (2-FAL, CO2+CO, ...) resultiert.

Eingangsgrößen für das thermo-hydraulische Alterungsmodell können z.B. u.a. die Umgebungstemperaturen während der betrachteten Betriebsdauer und die Last sein. In zweckmäßiger Ausgestaltung bilden ferner die Schalterstellung, Kühlungsstufe und/oder die vorliegende Spannung Eingangsgrößen für das thermo-hydraulische Alterungsmodell.

Es kann vorgesehen sein, dass in Schritt S3 bei der Berechnung der Alterungsproduktmengen für die verschiedenen Bereiche der festen Isolationsanordnung jeweils der Gleichgewichtszustand insbesondere des oder des jeweiligen Alterungsprodukts zwischen fester Isolationsanordnung und Isoliermedium berücksichtigt wird, bevorzugt unter Berücksichtigung der Zunahme des oder des jeweiligen Alterungsproduktes durch Alterung und der Durchmischung des oder des jeweiligen Alterungsproduktes im insbesondere gesamten Isoliermedium. Dies insbesondere, um die Aufnahme des wenigstens einen Alterungsprodukts in das Isoliermedium zu bestimmen.

Mit anderen Worten kann die Berechnung der Alterungsproduktmengen an lokalen Bereichen bzw. Teilen der festen Isolationsanordnung erfolgen, wo der Gleichgewichtszustand der Substanz bzw. des Alterungsproduktes zwischen lokaler fester Isolationsanordnung und lokalem Isoliermedium berücksichtigt wird, unter Berücksichtigung der Zunahme des oder des jeweiligen Alterungsproduktes durch Alterung und der Durchmischung des oder des jeweiligen Alterungsproduktes im insbesondere gesamten Isoliermedium.

Es kann also ein Ausgleichsvorgang berücksichtigt werden, insbesondere die Durchmischung/Umverteilung durch den Transport im Isoliermedium. Die Berechnung des lokalen Ausgleichs zwischen lokaler fester Isolationsanordnung bzw. Bereichen/Stellen dieser und lokalem Isoliermedium wird insbesondere durch zwei Effekte bestimmt: der Zunahme des bzw. des jeweiligen Alterungsprodukts durch Alterung und der Änderung durch den Transport im flüssigen bzw. gasförmigen Isoliermedium, wobei sich anstelle der iterativen Änderung durch den Transport eine Vereinfachung durch eine sofortige Übertragung ins gesamte Isoliermedium ergibt, wodurch z.B. der im Hotspot absorbierte spezifische Alterungsproduktwert nicht der hohen spezifischen Erzeugungsrate des Alterungsprodukts entsprechen muss, umgekehrt Bereiche/Stellen/Teile mit geringer Alterung angehobene Alterungswerte erhalten, als deren Alterung entspricht.

Aus der Berechnung lokaler Alterungsprodukt-Gleichgewichte kann ein integraler Wert gebildet werden. Das Alterungsmodell ist bevorzugt entsprechend ausgebildet.

Zweckmäßigerweise gilt, dass der Gleichgewichtszustand zumindest grob bekannt ist und entsprechend berücksichtigt wird. Im einfachsten Ansatz kann für die Aufteilung zwischen fester Isolation und Isoliermedium ein konstanter, temperaturunabhängiger Verhältniswert eingesetzt werden, indem der Dampfdruck in fester Isolation und Isoliermedium dieselbe Temperaturabhängigkeit haben, sodass deren Verhältnis nicht temperaturabhängig ist. Diese vereinfachte Annahme ist beim Dampfdruck vom in Zellulose und Öl gelösten Wasser nicht gegeben. Dort verschiebt sich die Feuchtigkeit bei steigender Temperatur in das Isoliermedium. Das zum Einsatz kommende thermo-hydraulische Alterungsmodell ist bzw. wird zweckmäßigerweise entsprechend ausgestaltet.

Alternativ oder zusätzlich kann bei der Berechnung der Alterungsproduktmengen in Schritt S3 eine Umverteilung der Alterungsprodukte von Stellen mit einer höheren Generationsrate zu Stellen mit einer niedrigeren Generationsrate, insbesondere über einen Transport durch das Isoliermedium, berücksichtigt werden. Auch hier gilt, dass das zum Einsatz kommende thermo-hydraulische Alterungsmodell zweckmäßigerweise entsprechend ausgestaltet ist bzw. wird. Dieser Effekt hängt insbesondere von den konkreten Löslichkeitseigenschaften des bzw. des jeweiligen Alterungsproduktes in den unterschiedlichen Bereichen der Isolationsanordnung und dem Isoliermedium ab.

In weiterer bevorzugter Ausgestaltung ist vorgesehen, dass in Schritt S3 die Berechnung der Alterungsproduktmengen unter Nutzung wenigstens einer Formel erfolgt, die auf Basis messtechnisch erfasster Daten erstellt wird oder wurde, insbesondere auf Basis von messtechnisch erfassten Daten, welche eine abnehmende DP-Zahl mit einer zunehmenden Menge wenigstens eines Alterungsproduktes, bevorzugt $CO_2$+CO und/oder 2-FAL, verknüpfen. 2-FAL steht dabei für 2-Furfural, eine in der Praxis besonders aussagekräftige Verbindung aus der Familie der Zellulose-Zersetzungsprodukte.

Es kann sich mit anderen Worten insbesondere um eine oder mehrere Formeln handeln, die unter Heranziehung von Messdaten erstellt wird bzw. wurde, die Alterungsgrößen einer festen Isolation, insbesondere DP-Zahlen, mit Mengen von wenigstens einem in ein zugehöriges Isoliermedium übergehenden Alterungsprodukt verknüpfen. Die DP-Zahl nimmt mit zunehmendem Alter ab und die Alterungsproduktmenge(n) zu.

Es sei angemerkt, dass auch andere Alterungsprodukte, wie z.B. Methanol, existieren. Diese sind aber in der Regel nicht stabil und werden weiter zersetzt. Die Verwendung dieser unstabilen Substanzen würde den Komplexitätsgrad also zusätzlich erhöhen. In allen Fällen ist am Ende aber CO2+CO vorzufinden. Generell hängt der Anteil von CO, der auf Sauerstoffarmut während des Zersetzungsprozesses weist, von den Zersetzungsbedingungen wie Temperatur und Schnelligkeit eines Temperaturanstiegs ab. Auch sind die Löslichkeitseigenschaften von CO in Öl schlechter als von CO2. Messtechnisch werden CO2 und CO zweckmäßigerweise getrennt erfasst.

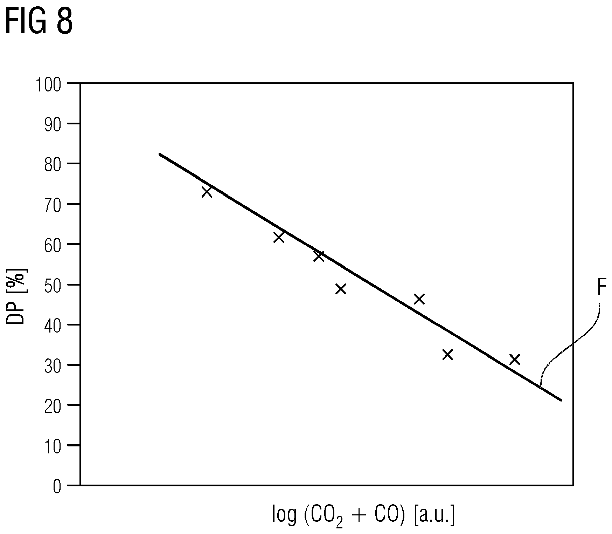

Rein beispielhaft sei genannt, dass an einem realen elektrischen Gerät, etwa an einem Transformator mit Ölbefülltem Tank und Zellulose-basierter fester Isolationsanordnung, bzw. an einem zugehörigen repräsentativen Messaufbau, insbesondere an mehreren beabstandeten Zeitpunkten sowohl die DP-Zahlen der festen Isolation(sanordnung) als auch Mengen von übergangenen Alterungsprodukten, wie $CO_2$+CO und/oder 2-FAL, im Öl messtechnisch erfasst werden bzw. wurden. Entsprechende Messdaten können beispielsweise in einem Graphen aufgetragen und wenigstens eine zugehörige Fit-Funktion ermittelt werden, die dann als die wenigstens eine Formel in das Simulationsmodell einfließen kann. Entsprechende Messdaten können extra für die Erstellung des Simulationsmodells erfasst werden oder worden sein. Alternativ oder zusätzlich kann auf vorhandene Messdaten zurückgegriffen werden. Rein beispielhaft sei auf den Artikel "Diagnosis of Thermal Degradation for Thermally Upgraded Paper in Mineral Oil" von Naoki Yamagata et al., 2008 International Conference on Condition Monitoring and Diagnosis, Beijing, China, April 21-24, 2008 verwiesen, welcher z.B. in Figur 8 einen Graphen zeigt, in dem für zwei verschiedene Papier-Arten die (mittlere) DP-Zahl (in Prozent vom Anfangswert) über der in das Öl übergehende Menge an $CO_2$+CO (ml/$g_{Papier}$) aufgetragen ist. Zugehörige Fit-Geraden sind ebenfalls eingezeichnet. Demnach liegen Labor-Messergebnisse für eine Korrelation von "$CO_2$+CO-Erzeugung pro Gramm Papier" zur DP-Zahl vor. Es handelt sich um Messwerte basierend auf einer Masse mit einer einzigen Temperatur (1-Massenmodell). Dies ist für ein elektrisches Gerät, wie einen Transformator, unzureichend. Im Rahmen dieser Ausführungsform der vorliegenden Erfindung fließen diese Messergebnisse in ein Netzwerkmodell ein, um die Komplexität eines elektrischen Gerätes, wie Transformators (Multi-Massenmodell, Ausgleichsvorgänge), zu beschreiben.

Für eine Korrelation zwischen DP-Zahl und 2-FAL, aufgenommen im Isoliermedium, z.B. Öl, kann beispielsweise eine modifizierte Version, insbesondere wenigstens eine modifizierte Gleichung, des sogenannten "De Pablo Modells" genutzt werden. Die Hauptgleichung dieses Modells ist gegeben durch

De Pablo:

$$[2FAL(\mu g/g\ Papier)] = ((10^6 * ((DP_0/DP_t) - 1))/162 * DP_0) * 96 * 0,3 * 1,2$$

Darin ist $DP_0$ die initiale DP-Zahl, DPt die DP-Zahl zu einem beliebigen Zeitpunkt während der Alterung, "g Papier" ist die Isolationsmasse, 162 das molekulare Gewicht von Glukoseeinheiten, 96 das molekulare Gewicht von Furfural, $10^6$ der Korrekturfaktor von g zu $\mu$g, 0,3 ist der Reaktionsertrag und 1,2 ist die Furfural-Absorptions-Korrektur von der festen Isolation.

Zu dem "De Pablo Modell" sei auch verwiesen auf das CIGRE Referenz Paper "The Condition of Solid Transformer Insulation at End-of-Life", CIGRE ELECTRA Nr. 321, April 2022, von Christoph Krause et al., darin insbesondere auf die Seite 4 und die dortigen Gleichungen (1) bis (3) nebst zugehöriger Erläuterungen.

In bevorzugter Weiterbildung des erfindungsgemäßen Verfahrens wird die vorstehende De Pablo Gleichung (entspricht Gleichung (3) auf Seite 4 des genannten CIGRE Referenz Papers), jedoch ohne den Faktor 1,2 am Ende genutzt, also

$$[2FAL(\mu g/g\ Papier)] = ((10^6 * ((DP_0/DP_t) - 1))/162 * DP_0) * 96 * 0,3,$$

um in Schritt S3 Mengen von 2-FAL als wenigstens einem Alterungsprodukt zu berechnen. Dies, da der Faktor 1,2 mangels transformatorspezifischer Information eine pauschale Umrechnung auf den DP-Wert im Hotspot darstellt.

Das Isoliermedium ist flüssig oder gasförmig. Das Isoliermedium kann ein Öl umfassen oder durch ein Öl gegeben sein, beispielsweise Mineralöl. Auch ein Gas als Isoliermedium ist möglich. Rein beispielhaft für ein Isoliergas, das als Isoliermedium vorgesehen sein bzw. betrachtet bzw. simuliert werden kann, sei SF6 genannt. Es ist auch möglich, dass ein Vakuum als (gasförmiges) Isoliermedium dient bzw. simuliert wird. Der Vollständigkeit halber sei angemerkt, dass auch unter Vakuumbedingungen ein Gasübertritt und Transport an andere Stellen, mit anderen Worten eine Umverteilung von Alterungsprodukten, möglich ist.

Die feste Isolationsanordnung kann ferner Zellulose, insbesondere Papier und/oder Pressboard, umfassen oder durch Zellulose, insbesondere Papier und/oder Pressboard, gegeben sein. Insbesondere Papier kann dabei in unterschiedlichen Qualitäten vorliegen, z.B. thermostabilisiert und nicht thermostabilisiert.

Bei anderen Materialien werden zweckmäßigerweise die materialabhängigen Eigenschaften labortechnisch ermittelt. Bei Aramid, einem Hochtemperatur-Kunststoff, ist z.B. die Bildung von CO2 oder anderen Zersetzungsprodukten, die als Aging-Marker geeignet sein könnten, mit heutigem Wissen kaum wahrnehmbar. Sehr wohl existieren Daten, um die Alterung an sich anhand der abnehmenden mechanischen Festigkeit abschätzen zu können.

Zellulose und Pressboard können auch andere Materialien zugemischt sein, insbesondere solche, die den Erhalt einer höheren Wärmeklasse gewährleisten.

Auch kann vorgesehen sein, dass unterschiedliche Bereiche der festen Isolationsanordnung, insbesondere verschiedene Teile dieser unterschiedlich ausgestaltet sind, sich z.B. durch verschiedene Materialqualitäten auszeichnen.

Anstelle der DP-Zahl von Zellulose könnte auch alternativ - ohne vorherige Bildung einer DP-Zahl - die Generation von Substanzen, die auch als Aging-Marker nutzbar sind, insbesondere Alterungsproduktmengen, auf Basis der Zeitinformation insbesondere eines Zeit-Profils berechnet werden, welches von mehreren Parametern abhängig sein kann, wie z.B. Temperatur, Feuchte, etc. [CO2+CO Messergebnisse versus Zeit: siehe das vorstehend genannte japanische Paper von Naoki Yamagata et al.].

Alterungsprodukte, wie z.B. CO2 und CO, können auch in dem Isoliermedium selbst erzeugt werden. In der Regel ist dieser Anteil deutlich geringer als der Beitrag aus der festen Isolation.

In Weiterbildung des erfindungsgemäßen Verfahrens kann vorgesehen sein, dass das elektrische Gerät einen mit dem Isoliermedium befüllten Tank umfasst, in welchem Komponenten des elektrischen Gerätes angeordnet sind, wobei eine oder mehrere der Komponenten mit der festen Isolationsanordnung versehen, insbesondere mit der festen Isolationsanordnung oder Teilen dieser umwickelt sind. Ein Transformator beispielsweise umfasst in der Regel mehrere in einem z.B. mit Öl befüllten Tank angeordnete Komponenten, die jeweils mit der/einer festen Isolation, z.B. Pressboard (Zellulose) und/oder Papier, versehen sind. Im Falle von Papier sind die betroffenen Komponenten insbesondere damit umwickelt. Rein beispielhaft für solche Komponenten seien Kern, Wicklungen und andere angrenzende Teile genannt.

Die insgesamt vorhandene feste Isolation kann also auch mehrere Teile bzw. Teilisolationen für die mehreren Komponenten umfassen, weshalb vorliegend auch von einer festen Isolationsanordnung gesprochen wird. Ist die feste Isolationsanordnung mehrteilig ausgebildet, können zwei oder mehr der Isolationsteile demnach auch beabstandet voneinander angeordnet sein.

Ist die feste Isolationsanordnung mehrteilig ausgebildet, können in Schritt S2 die verschiedenen Bereiche der festen Isolationsanordnung verschiedene Teile der festen Isolationsanordnung umfassen oder dadurch gegeben sein. Bevorzugt sind dann verschiedenen Komponenten des elektrischen Gerätes verschiedene Teile der festen Isolationsanordnung zugeordnet. Es kann insbesondere sein, dass verschiedene Komponenten des elektrischen Gerätes mit verschiedenen Teilen der festen Isolationsanordnung versehen, z.B. auch umwickelt sind.

Beispielsweise werden in Schritt S2 lokale Temperaturen und insbesondere lokale Alterungsgrößen, wie DP-Zahlen, für verschiedene Teile der festen Isolationsanordnung berechnet, etwa für den Teil der festen Isolationsanordnung, der sich an den Wicklungen befindet, für einen Isolationsteil wenigstens eines Abstandshalters, den Isolationsteil wenigstens eines Druckrings und den Isolationsteil wenigstens einer Montageplatte.

Die Alterungsgrößen sind solche, die einen Alterungsprozess der Isolation repräsentieren bzw. mit einem Alterungsprozess der Isolation in Verbindung stehen, sich insbesondere aufgrund wenigstens eines auftretenden Alterungsprozesses ändern. Es kann mit anderen Worten eine die fortschreitende Alterung der Isolation repräsentierende bzw. anzeigende Größe sei. Bei Zellulose ist dies z.B. die mit fortschreitender Zeit, also zunehmendem Alter abnehmende DP-Zahl, die vorstehend näher beschrieben wurde.

In Schritt S2 können bei der optionalen Berechnung der Alterungsgrößen für die verschiedenen Bereiche/Teile der festen Isolation bevorzugt neben der Temperaturverteilung weitere alterungsbestimmende Einflussgrößen berücksichtigt werden, insbesondere eine Feuchteverteilung. Beispielsweise können für verschiedene Bereiche/Teile insbesondere der festen Isolationsanordnung des elektrischen Gerätes lokale Temperaturen und lokale Feuchtewerte berechnet werden. Es wird also der Einfluss der Feuchte auf die Alterung mit in Betracht gezogen.

In Schritt S2 können - unter Berücksichtigung der lokalen Temperaturen und insbesondere Feuchten - Alterungsgrößen berechnet werden. In Weiterbildung werden für die verschiedenen Bereiche/Teile jeweils mehrere DP-Zahlen als die Alterungsgrößen für einen vorgegebenen Zeitraum ermittelt. Mit anderen Worten werden bevorzugt die mit fortschreitender Zeit und daher zunehmendem Alter der festen Isolationsanordnung abnehmenden DP-Zahlen ermittelt, dies insbesondere für verschiedene Bereiche/Teile der festen Isolationsanordnung. Besonders bevorzugt werden in Schritt S2 Alterungsgrößen, insbesondere DP-Zahlen, für einen Zeitraum von mehreren Stunden oder Tagen oder mehreren Monaten bzw. auch mehreren Jahren berechnet.

Auch kann vorgesehen sein, dass im Rahmen der Simulation bzw. des thermo-hydraulischen Alterungsmodells Teile der festen Isolation vereinfacht als Blöcke angenommen werden.

Im Laufe der technischen Weiterentwicklung nimmt der Trend zum Mischen unterschiedlicher Isolationsqualitäten zu. So ist der Einsatz von thermostabilisierten Papieren in der Wicklungsisolation mit ihren thermischen Hotspots in der US-Industrie mehr als 30 Jahre Stand der Technik. Allerdings wird in den gängigen Assessmentverfahren nicht darauf Rücksicht genommen, dass diese Zellulosequalität ein vollkommen geändertes Verhalten in der 2-FAL Erzeugung gegenüber der großen Restmasse an nicht-thermostabilisierten Zellulose einnimmt. Die 2-FAL Bildung bei Alterung ist extrem reduziert. Der Trend zur Vermischung unterschiedlicher Materialien, wie z.B. Aramid, nimmt zu, um höhere thermischen Wärmeklassen als der von Zellulose zu nutzen - siehe auch IEC 60076-14:2014. Da wird der Einsatz von empirischen Anpassfaktoren immer ungünstiger. Die erfindungsgemäße Verwendung eines Netzwerkmodells zur Berücksichtigung lokal individueller Materialeigenschaften ist auch hier besonders vorteilhaft.

Eine weitere Ausführungsform des erfindungsgemäßen Verfahrens zeichnet sich dadurch aus, dass eine Simulation des Betriebes für einen Simulationszeitraum von mehreren Monaten, insbesondere mehreren Jahren erfolgt. Mit anderen Worten wird im Rahmen des erfindungsgemäßen Verfahrens eine Langzeitsimulation für das elektrische Gerät durchgeführt. Es sei angemerkt, dass die für die Durchführung einer solchen Simulation benötigte tatsächliche Rechenzeit ("Echtzeit") die Zeitspannen, welche die Simulation abdeckt (Simulationszeit), natürlich erheblich unterschreiten kann und in der Regel erheblich unterschreiten wird.

Ergänzend sei auch vermerkt, dass im Falle von extremen Überlasten bzw. extrem schnell sich ändernden Lasten, auch eine Simulation für kurze Zeiten, z.B. der Größenordnung von Stunden, von großem Interesse sein kann. Hier ist die Gefahr von Bubbling, der Bildung von Gasblasen im Hochspannungsbereich, besonders groß.

Ein weiterer Gegenstand der vorliegenden Erfindung ist ein Computerprogramm umfassend Programmcode-Mittel, die bei der Ausführung des Programms auf wenigstens einem Computer den wenigstens einen Computer veranlassen, die Schritte des erfindungsgemäßen Verfahrens durchzuführen.

Das Computerprogramm kann ausgebildet sein, einen oder mehrere Werte von an dem elektrischen Gerät messtechnisch erfasste Alterungsproduktmengen entgegenzunehmen, um einen Vergleich dieser mit den in Schritt S3 berechneten Alterungsproduktmengen vorzunehmen.

Darüber hinaus betrifft die Erfindung ein computerlesbares Medium, das Instruktionen umfasst, die, wenn sie auf wenigstens einem Computer ausgeführt werden, den wenigstens einen Computer veranlassen, die Schritte des erfindungsgemäßen Verfahrens durchzuführen.

Bei dem computerlesbaren Medium kann es sich beispielsweise um eine CD-ROM oder DVD oder einen USB- oder Flashspeicher handeln. Es sei angemerkt, dass unter einem computerlesbaren Medium nicht ausschließlich ein körperliches Medium zu verstehen sein soll, sondern ein solches beispielswiese auch in Form eines Datenstromes und/oder eines Signals, welches einen Datenstrom repräsentiert, vorliegen kann.

Weitere Merkmale und Vorteile der vorliegenden Erfindung werden anhand der nachfolgenden Beschreibung unter Bezugnahme auf die beiliegende Zeichnung deutlich. Darin ist

Figur 1 eine rein schematische Teildarstellung eines Transformators,

Figur 2 eine rein schematische Darstellung eines thermo-hydraulischen Alterungsmodells für den Transformator aus Figur 1,

Figur 3 einen Graphen, in dem die normalisierte relative Alterungsrate über den Feuchtigkeitsgehalt in Zellulose aufgetragen ist,

Figur 4 ein Umgebungstemperaturprofil für ein Jahr,

Figur 5 einen Graphen, in dem lokale Temperaturen für verschiedene Bereiche des Transformators aus Figur 1 über einen Tag aufgetragen sind,

Figur 6 einen Graphen, in dem die Feuchte für verschiedene Bereiche des Transformators aus Figur 1 über einen Tag aufgetragen ist,

Figur 7 einen Graphen, in dem lokale DP-Werte für verschiedene Bereiche bzw. Teile der festen Isolationsanordnung des Transformators aus Figur 1 über der Zeit in Jahren aufgetragen sind, und

Figur 8 einen Graphen, in dem DP-Zahlen über der Menge an $CO_2$+CO aufgetragen sind.

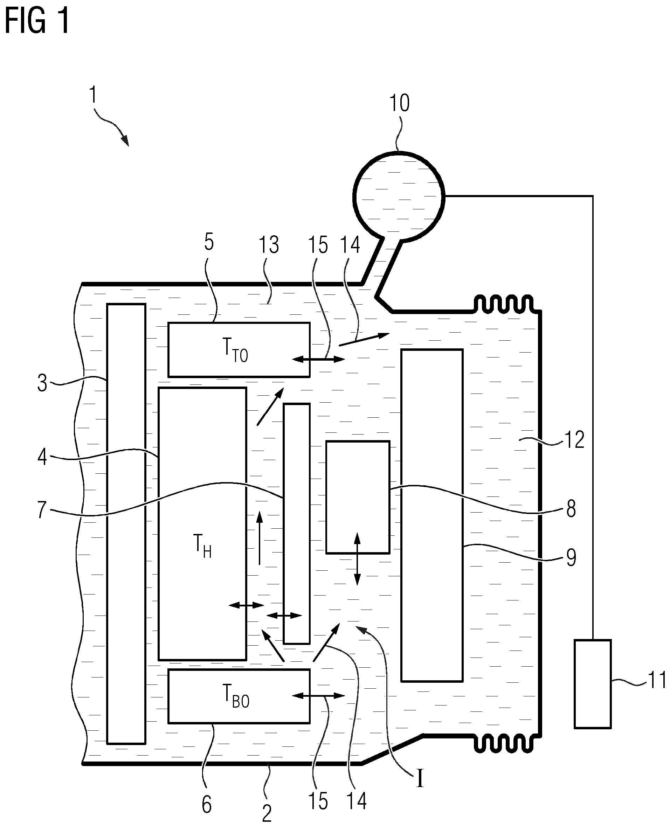

Die Figur 1 zeigt eine stark vereinfachte, rein schematische Teildarstellung eines durch einen Transformator 1 gegebenen elektrischen Gerätes.

Der Transformator 1 umfasst einen in der Figur nur abschnittsweise dargestellten Tank 2, in dem mehrere Komponenten des Transformators 1 angeordnet sind. Hiervon sind in der Figur 1 u.a. ein Kern 3, eine Wicklungsanordnung 4, ein Pressring 5, eine Montageplatte 6 sowie ein Wicklungszylinder 7 zu erkennen. Der Transformator 1 weist eine feste Isolationsanordnung I auf, die vorwiegend Pressboard und Papier, also Zellulose, umfasst bzw. daraus besteht und mehrteilig ausgebildet ist. Als Beispiel wurde die weit verbreitete Kernbauweise eines Leistungstransformators gewählt, dessen Aktivteil die sich um den Eisenkern 3 erstreckende Wicklungsanordnung 4 umfasst, deren Leiter durch Pressboardelemente (Zellulose) isoliert sind, wobei die Leiter typischerweise mit Papieren (Zellulose) umwickelt sind. Einerseits sind die zu der Wicklungsanordnung 4 gehörigen Pressboardelemente und Papiere Bestandteil der festen Isolationsanordnung I. Gleiches gilt für den Pressring 5, die Montageplatte 6 sowie den Wicklungszylinder 7, die jeweils aus einem oder mehreren Pressboardelementen bestehen.

Das mit dem Bezugzeichen 8 versehene Blockelement in Figur 1 steht repräsentativ für weitere Bereiche bzw. Teile der Isolationsanordnung I, die vorhanden sein können bzw. vorliegend vorhanden sind, insbesondere weitere Zellulose, auch für/an weitere(n) Komponenten des Transformators 1 außerhalb des Aktivteils. Sämtliche Papier- bzw. Pressboardteile , die sich an bzw. auf den verschiedenen Komponenten befinden, bilden zusammen die feste Isolationsanordnung I.

Mit 9 ist ein im Tank 2 vorhandener "Leerraum", mit anderen Worten eine Aussparung bzw. ein Loch angedeutet. Weiterhin schematisch dargestellt sind ein Konservator, mit anderen Worten Ölausdehnungsgefäß 10, ein Luftentfeuchter 11 und ein Kühler 12. Der Leerraum 9 liegt zwischen dem Kühler 12 und dem verbleibenden Bereich des Tanks 2. Es gibt Dehngefäße in offener Bauart, welche den Zutritt von Luft zulassen. Doch nimmt der Trend zu geschlossenen Systemen - z.B. mittels Gummimembran - zu, um den Zutritt von alterungsbeschleunigendem Sauerstoff zu verhindern.

Der Tank 2 ist bei dem dargestellten Ausführungsbeispiel ferner mit einem vorliegend flüssigen Isoliermedium befüllt, welches durch ein Mineralöl 13 gegeben ist. Man kann auch von einem ölisolierten Transformator 1 sprechen. Die feste Isolationsanordnung I und das Öl 13 bilden zusammen ein Isolationssystem des Transformators 1.

Die feste Isolationsanordnung I steht in Kontakt mit dem Isolieröl 13. Substanzen, die aufgrund der Alterung der festen Isolationsanordnung I entstehen, in das Isoliermedium 13 übergehen und dort detektiert werden können, werden auch als "Aging-Marker" bezeichnet. Rein beispielhaft für solche "Aging-Marker" seien $CO_2$+CO und/oder 2-FAL genannt.

Der Ölfluss ist in Figur 1 durch einfache Pfeile angedeutet, die beispielhaft mit der Bezugsziffer 14 versehen sind.

Doppelpfeile 15 deuten ferner rein schematisch den Feuchtigkeitsaustausch zwischen fester Isolationsanordnung I und flüssigem Isoliermedium 13 an.

Unter Durchführung des im Folgenden beschriebenen Ausführungsbeispiel des erfindungsgemäßen Verfahrens wird eine verbesserte Bewertung des Alterungszustandes und somit eine bessere Zustandsüberwachung des Betriebs des Transformators 1 möglich.

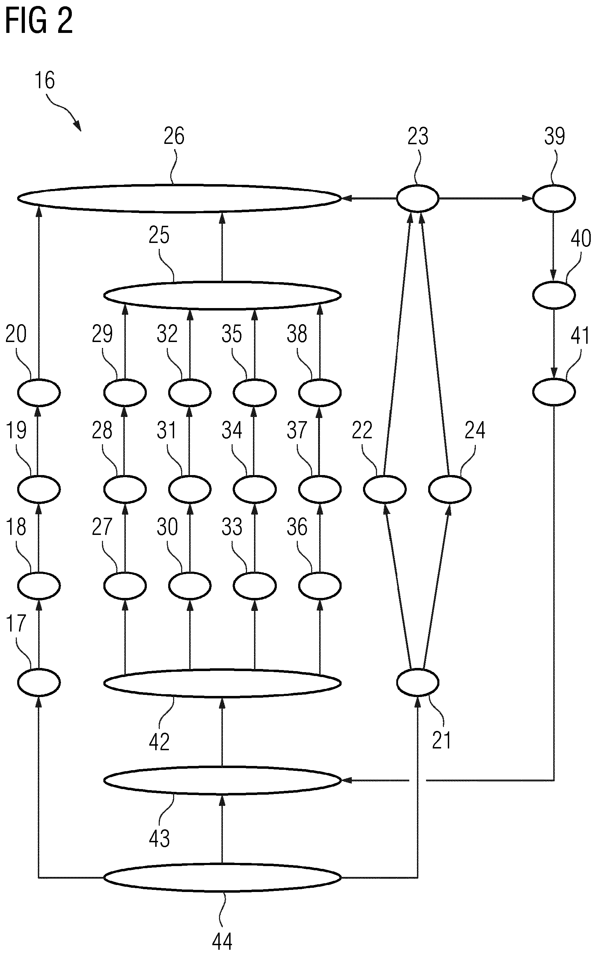

Dabei wird in Schritt S1 ein thermo-hydraulisches Alterungsmodell (kurz: THAM) 16 (siehe Figur 2) für den Transformator 1 bereitgestellt und/oder erstellt und mit dem Alterungsmodell 16 wird eine Simulation für den Transformator 1 durchgeführt, insbesondere für einen Simulationszeitraum von mehreren Monaten bzw. Jahren. Dabei werden innere Abläufe bzw. Vorgänge in dem Transformator 1 simuliert. Die Simulation kann in an sich bekannter Weise unter Nutzung wenigstens eines Computers erfolgen.

Vorliegend kommt eine erweiterte Version desjenigen thermo-hydraulischen Alterungsmodells zum Einsatz, welches in dem Artikel "Simulation of long-term transformer operation with a dynamic thermal, moisture and aging model", 5th International Colloquium on Transformer Research and Asset Management, October 2018, Opatija, Croatia , offenbart ist.

Die verschiedenen Komponenten bzw. Aspekte des in Figur 1 dargestellten Transformators 1 einschließlich des Ölflusses werden durch verschiedene Zweige (englisch: branches) mit spezifischen Eigenschaften, die durch Knoten (englisch: nodes) verbunden sind, modelliert, um ein thermisches Netzwerkmodell zu erhalten. In Figur 2 ist eine beispielhafte Anordnung von Zweigen und Knoten für die Haupt-Komponenten des Transformators 1 gezeigt. Dabei repräsentieren die Knoten 17-20 den Kern 3, die Knoten 21-23 den Großteil des Öls 13 im Tank 2 ("tank bulk oil"), die Knoten 21, 24 und 23 repräsentieren die Wand des Tanks 2, der Knoten 25 repräsentiert den Zusammenfluss des Öls 13 aus den darunterliegenden Komponenten, der Knoten 26 das Öl 13 im oberen Bereich des Tanks 2 ("Topoil" - das resultierende Heißöl) und die Membran des Konservators 10, der Knoten 23 das Öl 13 im oberen Bereich des Tanks 2 inklusive der Dichtungen, die Knoten 27-38 die Transformator-Wicklungen und die Knoten 39-41 das Kühlerelement. Die Knoten 42 bis 44 zeigen beispielhaft eine spezielle Konstruktion der Ölzuführung zu den Wicklungen der Wicklungsanordnung 4. Die Zweige sind in Figur 2 durch Pfeile angedeutet, welche die Knoten 17-44 verbinden.

Im Rahmen der Simulation mit dem Alterungsmodell 16 werden in Schritt S2 für verschiedene Bereiche des elektrischen Gerätes 1, hier für verschiedene Teile der festen Isolationsanordnung I und insbesondere Bereiche des Öls 13, lokale Temperaturen berechnet, die sich über die aus Strom und Spannung erzeugten elektrischen Verluste zeitverzögert, also dynamisch, einstellen.. In der Figur 1 ist angedeutet, dass der Pressring 5 die Temperatur $T_{TO}$ des Öls 13 im oberen Bereich des Tanks 2 ("Topoil") aufweist, die Wicklungsanordnung 4 mit den Wicklungen und Pressboard-Abstandshaltern die Hotspot-Temperatur $T_H$ und die Montageplatte 6 die Temperatur $T_{BO}$ des Öls 13 im unteren Bereich ("Bottom Oil").

In Schritt S2 werden ferner für verschiedene Bereiche der festen Isolationsanordnung I, hier für verschiedene Isolationsteile lokale Alterungsgrößen, konkret lokale DP-Zahlen, berechnet. Dabei erfolgt die Berechnung unter Berücksichtigung der ermittelten lokalen Temperaturen und weiterer alterungsbestimmender Einflussgrößen, nämlich dem Sauerstoff und der lokalen Feuchte. Die bei dem hiesigen Ausführungsbeispiel umfasste Berechnung lokaler Alterungsgrößen ist optional.

In Schritt S3 werden für die verschiedenen Bereiche/Teile der Isolationsanordnung I Mengen von wenigstens einem Alterungsprodukt, das aufgrund der Alterung der festen Isolationsanordnung I entsteht und in das Isoliermedium 13 übergeht, berechnet, dies vorliegend unter Berücksichtigung der in Schritt S2 erhaltenen lokalen DP-Zahlen, und insbesondere Massen der Isolationsanordnung I.

Das thermo-hydraulische Alterungsmodell 16 ist dabei dazu ausgebildet, folgende Größen in Bezug auf die Feuchte und das Alterungsverhalten zu berechnen:

- Den Feuchtigkeitsaustausch zwischen fester Isolationsanordnung I und Isoliermedium 13, einschließlich eines möglichen Feuchtigkeitsaustauschs mit der Atmosphäre.
- Berechnung der lokalen Alterungsgrößen, insbesondere des Polymerisationsgrades (DP-Wert), von verschiedenen Bereichen/Teilen der Zelluloseisolation I, insbesondere dem Hotspot unter Berücksichtigung von:

  ◦ dem Einfluss von Feuchtigkeit und Sauerstoff
  ◦ der Zellulosequalität (z.B. nicht thermostabilisiertes oder thermostabilisiertes Papier)
  ◦ der Feuchtigkeitsbildung durch die Alterung selbst

- Einfluss der Alterung (DP-Wert) auf die Feuchtigkeitsaufnahme der Zellulose, und
- Risiko von Blasenbildung, wenn der Druck von gelösten Gasen und Feuchtigkeit im Isoliermedium 13 den darauf lastenden Umgebungsdruck übersteigt. Dabei sollte dieser Druck bzw. die Temperatur zusätzlich etwas erhöht sein, um die Grenzflächenspannung der Flüssigkeit zur Bildung einer Gasblase im Isolieröl 13 überwinden zu können.

Alterung führt zu einer Verschlechterung der mechanischen Festigkeit der Zellulose bzw. der Zugfestigkeit des Papiers. Ein Absinken der Zugfestigkeit auf unter 40 % des Ausgangswertes wird häufig als riskanter Zustand hinsichtlich der Kurzschlussfestigkeit angesehen. Anstelle der gemessenen Zugfestigkeit ist die durchschnittliche Länge der Zellulosefasern, der Polymerisationsgrad (DP-Wert bzw. DP-Zahl), eine gängige Größe zur Beschreibung des Zustands der Zellulose. Zugfestigkeiten von etwa 40 % korrelieren mit DP-Werten von etwa 200. Neue Transformatoren weisen in der Regel DP-Zahlen von über 900 auf. Das Alterungsmodell 16 berechnet die Abnahme des DP-Wertes in jedem modellierten festen Isolierteil 4-8 des Transformators 1 gemäß Figur 1.

Für die Berechnung der lokalen DP-Zahlen wird bevorzugt folgende Gleichung genutzt:

$$DP_t = \left[ DP_{t-1}^{1-p(T)} + O_t \cdot M_t \cdot \Delta t \cdot (p(T) - 1) \right]^{\frac{1}{1-p(T)}}$$

Darin ist $M_t$ ein Feuchtigkeitsfaktor, $p(T)$ eine Temperaturfunktion und $O_t$ ein Sauerstoff-Faktor, welche den DP-abfall gemäß der Gleichung stark beeinflussen.

Für die Temperaturfunktion gilt:

| Nicht thermostabilisiertes Papier und Zellulose | Thermostabi-lisiertes Papier |
|---|---|
| $p(T) = 1{,}182455 \cdot e^{0{,}005791 \cdot T}$ | $p(T) = 1{,}024508 \cdot e^{0{,}006021 \cdot T}$ |

Für den Sauerstoff-Faktor wird folgende Gleichung genutzt:

$$O(T, ppm) = 1 + (O_T - 1) \cdot O_{ppm}/O_{sat} \; ,$$

mit $O_{sat}$=37000 ppm (< 1.000 m Meeresniveau, in Mineralöl) und $O_{ppm}$ gleich der im Transformator vorliegenden Sauerstoffkonzentration.

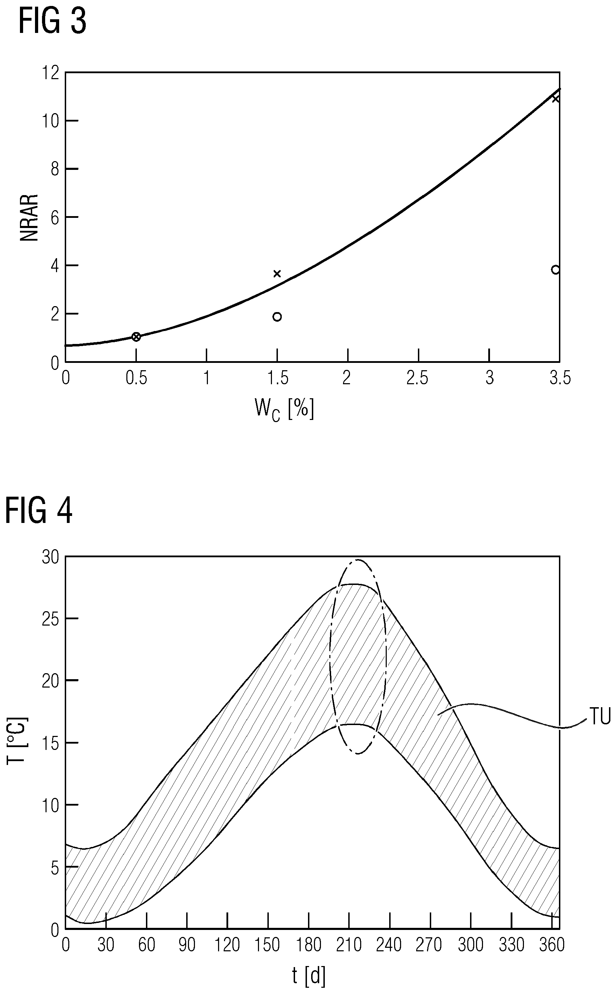

Der Graph gemäß Figur 3 zeigt ferner den Einfluss der Feuchtigkeit auf die relative Alterungsrate. Aufgetragen ist die normalisierte relative Alterungsrate (Normalized Relative Aging Rate, NRAR - $M_t$-Faktor normalisiert auf 1 bei 0,5% Feuchtigkeit) über der Feuchtigkeit in Zellulose $W_C$ in %. Die durchgezogene Linie entspricht dabei mit dem Alterungsmodell 16 berechneten Werten, während die Kreuze und Punkte Werte gemäß IEC 60076-7:2018 darstellen, die Kreuze für nicht thermostabilisiertes Papier und die Punkte für thermostabilisiertes Papier. Wie man erkennt, steigt die normalisierte relative Alterungsrate mit zunehmender Feuchtigkeit der festen Isolationsanordnung I, hier Zellulose, an.

Der Graph aus Figur 4 zeigt das im Rahmen der Simulation verwendete bzw. zugrunde gelegte Profil der Umgebungstemperatur für ein Jahr. Es wurde für jedes Simulationsjahr das gleiche, in dieser Figur dargestellte Umgebungstemperaturprofil genutzt.

Zusätzlich wird zweckmäßigerweise die Last, Spannung und Schalterstellung berücksichtigt. Sofern mehrere Kühlstufen vorhanden sind, können diese definiert werden (z.B. Ölpumpen, Lüfter)

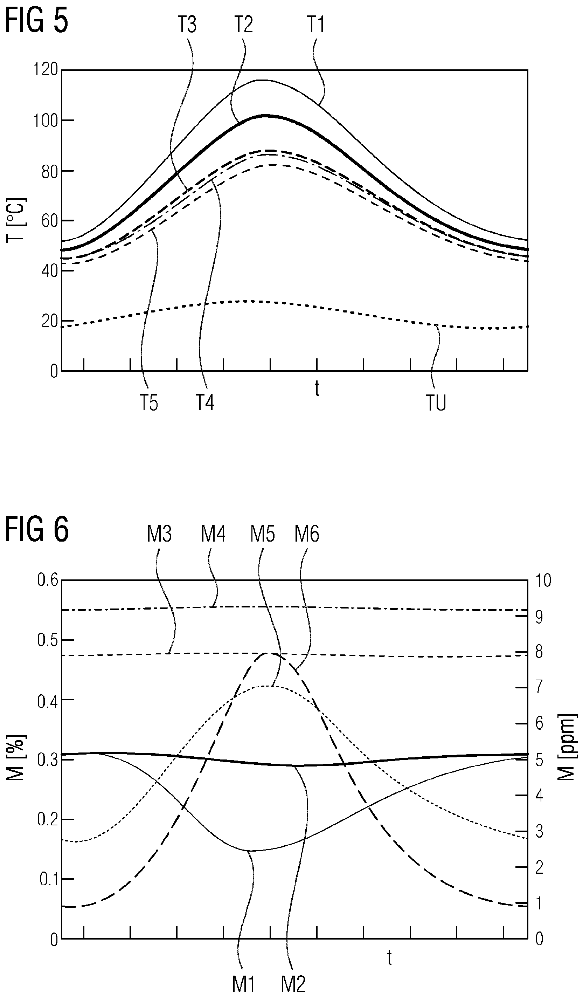

Der Graph gemäß Figur 5 zeigt mit dem Alterungsmodell 16 berechnete Temperaturen für verschiedene Teile der Isolationsanordnung I und Bereiche des Öls 13. Die Temperaturen sind über der Zeit aufgetragen, wobei die X-Achse hier einem beispielhaften Tag entspricht, konkret dem heißesten Tag des Jahres, der in Figur 4 markiert ist.

Bei den lokalen Temperaturen aus Figur 5 handelt es sich um:

T1    die Temperatur des Papiers im HotSpot,
T2    die Temperatur der Abstandshalter beim HotSpot
T3    die Temperatur des Öls 13 oben am Wicklungsdruckring
T4    die Temperatur des Heissöls im Kühler 12 (oben)
T5    die Temperatur die Temperatur des abgekühltem Öls 13 (unten) beim Eintritt unter der Wicklungsanordnung 4
TU    die Umgebungstemperatur

Figur 6 zeigt den berechneten lokalen Feuchtigkeitsgehalt für verschiedene Bereiche/Teile für den beispielhaften, heißesten Tag, nämlich:

M1    die Feuchtigkeit der Papierumwicklung im Hotspot
M2    die Feuchtigkeit der Abstandshalter beim Hotspot
M3    die Feuchtigkeit des Wicklungsdruckrings 5 (über den Wicklungen)
M4    die Feuchtigkeit der Wicklungsmontageplatte 6 (unter den Wicklungen)
M5    die Wasseraktivität bei Kaltöltemperatut T_bottom
M6    der Feuchtegehalt im Mineralöl 13 in ppm

Es sei angemerkt, dass die Skala der Y-Achse auf der linken Seite den Feuchtigkeitsgehalt von Zellulose in % sowie den Feuchtigkeitsgehalt von Öl ausgedrückt als Wasseraktivität (aw) in % zeigt - das ist der relative Feuchtedampfdruck, der sich auf den Dampfdruck von Wasser bei selber Temperatur bezieht, während die der rechten Y-Achse die Feuchtigkeit von Öl in ppm wiedergibt.

Im Rahmen der Simulation werden mit dem Alterungsmodell 16 für jeden Tag entsprechende, in den Figuren 5 und 6 aufgetragene Werte berechnet.

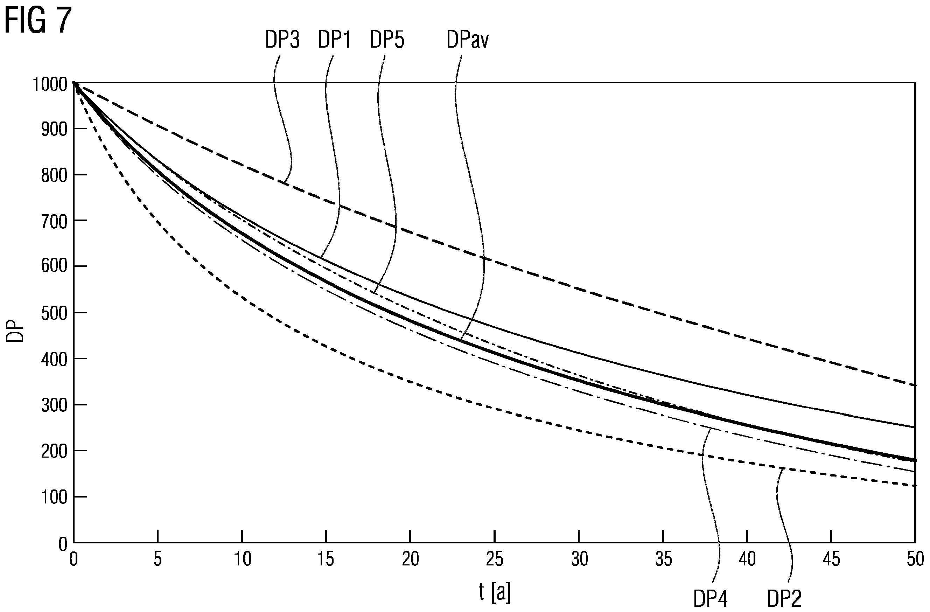

Die Figur 7 zeigt in Schritt S2 mit dem Alterungsmodell 16 (optional) berechnete lokale DP-Zahlen DP1-DP5 für die verschiedenen festen Isolationsteile 4-8 des Transformators 1 aus Figur 1 aufgetragen über der Zeit in Jahren. Wie man erkennt, ist hier ein Simulationszeitraum vom 50 Jahren gewählt worden, wobei dies rein beispielhaft zu verstehen ist. Mit anderen Worten ist die Abnahme der lokalen DP-Zahlen DP1-DP5 für einen Zeitraum von 50 Jahren simuliert. Auffällig an diesem Beispiel ist, dass durch die Wahl von thermostabilisiertem Papier in den Wicklungen deren Alterung geringer ausfallen kann als an anderen Isolierteilen.

Bei den lokalen DP-Zahlen aus Figur 7 handelt es sich konkret um:

DP1    DP-Zahlen der Papierumwicklung im Hotspot
DP2    DP-Zahlen de Abstandshalter beim Hotspot
DP3    DP-Zahlen der Papierumwicklung, über eine Wicklung gemittelt
DP4    DP-Zahl im Wicklungsdruckring 5 (über den Wicklungen)
DP5    DP-Zahl in der Wicklungsmontageplatte 6 (unter den Wicklungen)
DPav    Durchschnittswert aller lokalen DP-Werte im Netzwerkmodell

Es sei angemerkt, dass anstelle der DP-Zahlen von Zellulose alternativ auch - ohne vorherige Bildung bzw. Berechnung der DP-Zahlen - die Generation von Substanzen, die auch als Aging-Marker nutzbar sind, auf Basis eines Zeit-Profils berechnet werden, welches von mehreren Parametern abhängig ist, wie z.B. Temperatur, Feuchte, etc. [$CO_2$+CO Messergebnisse versus Zeit: siehe das vorstehend genannte japanische Paper von Naoki Yamagata et al.].

Wie vorstehend angemerkt, werden in Schritt S3 - bei dem hier beschriebenen Ausführungsbeispiel unter Berücksichtigung der lokalen Alterungsgrößen, hier lokalen DP-Zahlen DP1-DP5 - Mengen von wenigstens einem Alterungsprodukt das aufgrund der Alterung der festen Isolationsanordnung I entsteht und in das Isoliermedium übergeht, berechnet. Vorliegend werden konkret Mengen von $CO_2$+CO und/oder Mengen von 2-FAL berechnet, wobei dies beispielhaft zu verstehen ist.

Dabei erfolgt die Berechnung der Mengen von $CO_2$+CO unter Nutzung wenigstens einer Formel, die auf Basis messtechnisch erfasster Daten erstellt wurde, hier auf Basis von messtechnisch erfassten Daten, welche eine abnehmende DP-Zahl mit einer zunehmenden Menge von $CO_2$+CO verknüpfen. Die Figur 8 zeigt rein beispielhaft einen zugehörigen Graphen mit entsprechenden Wertepaaren und einer zugehörigen Fit-Funktion F. Es sei betont, dass die Figur 8 nur rein schematischer Natur ist und der bloßen Veranschaulichung dient. Die X-Achse ist unskaliert und einheitenlos dargestellt und kann - wie im vorliegenden Fall - beispielsweise auch logarithmisch sein.

Es kann beispielswiese auf konkrete Messergebnisse zurückgegriffen werden, wie sie in dem Artikel "Diagnosis of Thermal Degradation for Thermally Upgraded Paper in Mineral Oil" von Naoki Yamagata et al., 2008 International Conference on Condition Monitoring and Diagnosis, Beijing, China, April 21-24, 2008, veröffentlicht sind, etwa in Figur 8 aus diesem Artikel. Alternativ oder zusätzlich können Messungen an Versuchsaufbauten und/oder Messungen an realen elektrischen Geräten durchgeführt werden, um Daten für eine Korrelation von DP-Zahlen und Mengen an $CO_2+CO$ zu erhalten.

Um alternativ oder zusätzlich Mengen von 2-FAL zu berechnen, wird bevorzugt die Gleichung

$$[2FAL(\mu g/g\ Papier)]= ((10^6*((DP_0/DP_t)-1))/162*DP_0)*96*0,3,$$

genutzt.

Bei der Berechnung der Alterungsproduktmengen wird deren Gleichgewichtszustand zwischen fester Isolationsanordnung I und Isoliermedium 13 berücksichtigt, insbesondere, um die Aufnahme des bzw. des jeweiligen Alterungsproduktes, hier des $CO_2+CO$ und/oder 2-FAL, in das Isoliermedium 13 zu bestimmen.

Es wird ferner eine Umverteilung des bzw. des jeweiligen Alterungsproduktes, hier von $CO_2+CO$ und/oder 2-FAL, von Stellen mit einer höheren Generationsrate zu Stellen mit einer niedrigeren Generationsrate über den Transport durch das Isoliermedium 13 berücksichtigt.

Dazu wird dem im Öl gelösten Alterungsprodukt "A" $g_A/g_{Öl}$ (g...Gramm) bzw. $\mu l_A/l_{Öl}$ (l...Liter) ein Gasdruck $p_{A,Öl}$ zugeordnet, der proportional zur Konzentration des Alterungsproduktes $g_A/g_{Öl}$ bzw. $\mu l_A/l_{Öl}$ ist. In der Zellulose wird dem Gasdruck eine Konzentration des Alterungsprodukts in der Zellulose zugeordnet, $g_A/g_{Zellulose} = F_{Zellulose} \cdot p_{A,Öl}$. Dieser Faktor $F_{Zellulose}$ ist zweckmäßigerweise an die Messergebnisse anzupassen, die sich aus dem Netzwerkmodell ergeben, wenn Laborergebnisse über die Absorptionsfähigkeit des Alterungsprodukts in der Zellulose fehlen. Im ersten Schritt wird eine Temperaturabhängigkeit der Absorptionseigenschaften vernachlässigt, da sie deutlich geringer als bei Feuchtigkeit ist. So wird im Netzwerkmodell 16 bei jedem iterativen Rechenschritt zuerst die durch Alterung zusätzlich erzeugte Menge des Alterungsprodukts berechnet und zum vorangegangenem Wert des in Zellulose gelösten Alterungsprodukts addiert wird, worauf in einem zweiten Schritt, aufgrund der Übertragung ins Öl, die Alterungsproduktmenge $g_A$ zwischen jedem einzelnen Isolierteil-Knoten und umgebenden Öl umverteilt wird, bis in jedem einzelnen Isolationsteil des Netzwerkmodells die Drücke in fester $p_{A,Zellulose}$ und flüssiger/gasförmiger Isolation $p_{A,Öl}$ gleich sind. Durch die Ölströmung, die in der iterativen Rechnung berücksichtigt wird, kommt es zu einer allmählichen Durchmischung des im Öl gelösten Alterungsprodukts. Bei geringer Temperaturabhängigkeit des Faktors $F_{Zellulose}$ und langsamer Zunahme der Alterungsproduktmenge gegenüber dem Durchmischen im strömenden Öl ist eine Vereinfachung gestattet, indem die einzelnen in Öl gelösten Alterungsproduktmengen sofort im gesamten Öl homogen aufgeteilt werden, wonach sich wie oben als zweiter Schritt beschrieben der Gleichgewichtszustand durch Verschieben der Alterungsproduktmenge mit den einzelnen Isolationsteilen (Knoten) einstellt. Das ist ein dynamischer Vorgang, da die einzelnen lokalen Parameter wie Temperatur meist "in Bewegung" sind. Sollte sich ein vorübergehender ausgeglichener stationärer Zustand ergeben, sind die Werte in allen einzelnen lokalen Ölvolumina identisch und der Austausch zwischen fester Isolationsanordnung und insbesondere flüssigem Isoliermedium ist zumindest kurzzeitig zum Stillstand gekommen, ehe nicht die Zunahme des Alterungsprodukts in der Zellulose sich im Öl wie oben beschrieben bemerkbar macht.

Wie oben angemerkt, stellt das im Rahmen des hier beschriebenen Ausführungsbeispiels verwendete thermohydraulische Alterungsmodell 16 eine erweiterte Version desjenigen Modells aus dem Artikel "Simulation of long-term transformer operation with a dynamic thermal, moisture and aging model", ", 5th International Colloquium on Transformer Research and Asset Management, October 2018, Opatija, Croatia dar. Die Erweiterung betrifft dabei die Berechnung der Mengen von $CO_2+CO$ und/oder 2-FAL auf die vorstehend beschriebenen Weisen. Die zusätzliche Erzeugung des Aging-Markers erfolgt für jeden einzelnen Punkt im Netzwerkmodell, in einem zweiten Schritt erfolgt eine Umverteilung über die Ausgleichsvorgänge über das Öl 13. Prinzipiell steht die Bewertung der thermisch am höchsten beanspruchten Teile, die sogenannten Hotspots in der Isolationsanordnung I im Mittelpunkt. Die Aging-Marker an den Hotspots stehen aber in Wechselwirkung, durch Ausgleichvorgänge bzw. Umverteilungen. Das Modell 16 ist entsprechend ausgebildet.

Die über die Simulation berechneten Mengen von $CO_2+CO$ und/oder 2-FAL werden anschließend bevorzugt herangezogen, um zwischen einem normalen und einem fehlerhaften Zustand des Transformators 1 zu unterscheiden. Hierfür werden die berechneten Mengen von $CO_2+CO$ und/oder 2-FAL jeweils aufsummiert, um jeweils einen Gesamtsimulationswert zu erhalten. Es werden ferner entsprechende Messergebisse von einem realen Transformator, für den der Transformator 1 aus Figur 1, auf dem die Simulation basiert, zweckmäßigerweise einen digitalen Zwilling darstellt, bereitgestellt bzw. von einem Computerprogramm, welches der Durchführung der Simulation dient, empfangen.

Insbesondere aus dem Vergleich zwischen Simulationsergebnis(sen) und Messwert(en) kann anschließend eine Entscheidung getroffen werden, ob ein normaler oder ein fehlerhafter Zustand des Transformators 1 vorliegt. In letzterem Falle kann eine Warnung ausgegeben werden. Auch falls keine Messung eines "Aging Markers" wie 2-FAL erfolgt, also keine entsprechenden Messergebnisse für einen solchen Vergleich vorliegen, erfolgt eine Bewertung des Alterungszustandes, da das Modell 16 eine Aussage über die kritische Alterung im Hotspot macht. Das unterstützt die Lebensdauerbetrachtung und somit rechtzeitige Einplanung von Neu-Investitionen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

Unabhängig vom grammatikalischen Geschlecht eines bestimmten Begriffes sind Personen mit männlicher, weiblicher oder anderer Geschlechteridentität mit umfasst.

## Patentansprüche

1. 5 Computerimplementiertes Verfahren zur Alterungsbeurteilung und Zustandsüberwachung eines eine feste Isolationsanordnung (I) und ein mit der festen Isolationsanordnung (I) in Kontakt stehendes flüssiges und/oder gasförmiges Isoliermedium (13) umfassenden elektrischen Gerätes (1), wobei das elektrische Gerät ein Transformator oder eine Drossel ist, bei dem

   S1) ein thermo-hydraulisches Alterungsmodell (16) des elektrischen Gerätes (1) bereitgestellt und/oder erstellt und mit dem Alterungsmodell (16) eine Simulation für das elektrische Gerät (1) durchgeführt wird,
   S2) im Rahmen der Simulation für verschiedene Bereiche des elektrischen Gerätes für verschiedene Bereiche der festen Isolationsanordnung (I) und/oder für verschiedene Bereiche des von dem Isoliermedium (13) eingenommenen Volumens, lokale Temperaturen (T1-T5) berechnet werden, wobei unter Berücksichtigung der lokalen Temperaturen und weiterer alterungsbestimmender Einflussgrößen, und zwar der lokalen Feuchte und/oder des Sauerstoffgehaltes des Isoliermediums (13), für verschiedene Bereiche der festen Isolationsanordnung (I) lokale Alterungsgrößen (DP1-DP5) berechnet werden,
   S3) für die verschiedenen Bereiche der festen Isolationsanordnung (I) unter Berücksichtigung der berechneten lokalen Temperaturen und/oder, sofern in Schritt S2 berechnet, der lokalen Alterungsgrößen (DP1-DP5) und unter zusätzlicher Berücksichtigung von Massen der festen Isolationsanordnung (I), Mengen von wenigstens einem Alterungsprodukt, das aufgrund der Alterung der festen Isolationsanordnung (I) entsteht und in das Isoliermedium (13) übergeht, berechnet werden, wobei die berechneten Alterungsproduktmengen herangezogen werden, um zwischen einem normalen und einem fehlerhaften Zustand des elektrischen Gerätes (1) zu unterscheiden.

2. Verfahren nach Anspruch 1,
   **dadurch gekennzeichnet, dass**
   ein Vergleich der in Schritt S3 berechneten Alterungsproduktmengen mit einer oder mehreren an dem elektrischen Gerät (1) messtechnisch erfassten Alterungsproduktmengen erfolgt und anhand des Vergleichsergebnisses zwischen einem normalen und einem fehlerhaften Zustand des elektrischen Gerätes (1) unterschieden wird.

3. Verfahren nach einem der Ansprüche 1 oder 2,
   **dadurch gekennzeichnet, dass**
   das thermo-hydraulische Alterungsmodell (16) als Netzwerkmodell ausgebildet ist.

4. Verfahren nach einem der vorhergehenden Ansprüche,
   **dadurch gekennzeichnet, dass**
   in Schritt S3 Mengen von 2-FAL und/oder $CO_2$+CO als Alterungsproduktmengen berechnet werden.

5. Verfahren nach einem der vorhergehenden Ansprüche,
   **dadurch gekennzeichnet, dass**
   in Schritt S3 bei der Berechnung der Alterungsproduktmengen für die verschiedenen Bereiche der festen Isolationsanordnung (I) jeweils der Gleichgewichtszustand des oder des jeweiligen Alterungsprodukts zwischen fester Isolationsanordnung (I) und Isoliermedium (13) berücksichtigt wird, unter Berücksichtigung der Zunahme des oder des jeweiligen Alterungsproduktes durch Alterung und der Durchmischung des oder des jeweiligen Alterungsproduktes im Isoliermedium (13), um die Aufnahme des wenigstens einen Alterungsproduktes in das Isoliermedium (13) zu bestimmen.

6. Verfahren nach einem der vorhergehenden Ansprüche,

**dadurch gekennzeichnet, dass**

in Schritt S3 bei der Berechnung der Alterungsproduktmengen eine Umverteilung des wenigstens einen Alterungsproduktes von Stellen mit einer höheren Generationsrate zu Stellen mit einer niedrigeren Generationsrate über einen Transport durch das Isoliermedium (13) berücksichtigt wird.

7. Verfahren nach einem der vorhergehenden Ansprüche,
**dadurch gekennzeichnet, dass**
in Schritt S3 die Berechnung der Alterungsproduktmengen unter Nutzung wenigstens einer Formel erfolgt, die auf Basis messtechnisch erfasster Daten erstellt wird oder wurde.

8. Verfahren nach einem der vorhergehenden Ansprüche,
**dadurch gekennzeichnet, dass**

in Schritt S3 Mengen von 2-FAL als Alterungsproduktmengen berechnet werden, und die Formel

$$[2FAL(\mu g/g \ Papier)] = ((10^6 * ((DP_0/DP_t) - 1))/162 * DP_0) * 96 * 0,3,$$

für die Berechnung genutzt wird, wobei $DP_0$ die initiale DP-Zahl, DPt die DP-Zahl zu einem beliebigen Zeitpunkt während der Alterung und "g Papier" die Masse der festen Isolationsanordnung (I) bzw. des jeweiligen Teiles der festen Isolationsanordnung (I) ist, wobei die feste Isolationsanordnung (I) Papier und/oder Pressboard umfasst oder durch Papier und/oder Pressboard gegeben ist.

9. Verfahren nach einem der vorhergehenden Ansprüche,
**dadurch gekennzeichnet, dass**
das Isoliermedium (13) ein Öl umfasst oder durch ein Öl gegeben ist, und/oder dass die feste Isolationsanordnung (I) Zellulose umfasst oder durch Zellulose gegeben ist.

10. Verfahren nach einem der vorhergehenden Ansprüche,
**dadurch gekennzeichnet, dass**
das elektrische Gerät (1) einen mit dem Isoliermedium (13) befüllten Tank (2) umfasst, in welchem Komponenten des elektrischen Gerätes (1) angeordnet sind, wobei eine oder mehrere der Komponenten mit der festen Isolationsanordnung (I) versehen sind.

11. Verfahren nach einem der vorhergehenden Ansprüche,
**dadurch gekennzeichnet, dass**
dass die feste Isolationsanordnung (I) mehrteilig ausgebildet ist und in Schritt S2 die verschiedenen Bereiche der festen Isolationsanordnung (I) verschiedene Teile der festen Isolationsanordnung (I) umfassen oder dadurch gegeben sind, wobei verschiedenen Komponenten des elektrischen Gerätes (1) verschiedene Teile der festen Isolationsanordnung (I) zugeordnet sind, wobei verschiedene Komponenten des elektrischen Gerätes (1) mit verschiedenen Teilen der festen Isolationsanordnung (I) versehen sind.

12. Verfahren nach einem der vorhergehenden Ansprüche,
**dadurch gekennzeichnet, dass**
eine Simulation des Betriebes für einen Simulationszeitraum von mehreren Stunden, Monaten oder Jahren erfolgt.

13. Computerprogramm umfassend Programmcode-Mittel, die bei der Ausführung des Programms auf wenigstens einem Computer den wenigstens einen Computer veranlassen, die Schritte des Verfahrens nach einem der Ansprüche 1 bis 12 durchzuführen.

14. Computerlesbares Medium, das Instruktionen umfasst, die, wenn sie auf wenigstens einem Computer ausgeführt werden, den wenigstens einen Computer veranlassen, die Schritte des Verfahrens nach einem der Ansprüche 1 bis 12 durchzuführen.

**EP 4 428 554 B1**

**Claims**

1. A computer-implemented method for assessing ageing and for monitoring a condition of an electrical device (1) comprising a solid insulation arrangement (I) and a liquid and/or gaseous insulation medium (13) that is contact with the solid insulation arrangement (I), wherein the electrical device is a transformer or a choke, in which

    S1) a thermo-hydraulic ageing model (16) of the electrical device (1) is provided and/or created and a simulation for the electrical device (1) is performed with the aging model (16),
    S2) local temperatures (T1-T5) are calculated for different regions of the electrical device for different regions of the solid insulation arrangement (I) and/or for different regions of the volume occupied by the insulation medium (13) as part of the simulation, wherein local ageing variables (DP1-DP5) are calculated for different regions of the solid insulation arrangement (I), taking into account the local temperatures and further influencing variables that determine ageing, in particular local humidity and/or oxygen content of the insulation medium (13),
    S3) quantities of at least one ageing product that originates due to the ageing of the solid insulation arrangement (I) and passes into the insulation medium (13) are calculated for the different regions of the solid insulation arrangement (I), taking into account the calculated local temperatures and/or, if calculated in step S2, the local ageing variables (DP1-DP5) and additionally taking into account masses of the solid insulation arrangement (I), wherein the calculated ageing product quantities are used to differentiate between a normal and a faulty condition of the electrical device (1).

2. The method according to claim 1,
   **characterised in that**
   a comparison of the ageing product quantities calculated in step S3 with one or more ageing product quantities metrologically acquired at the electrical device (1) takes place and a normal and a faulty condition of the electrical device (1) are differentiated based on the comparison result.

3. The method according to any one of claims 1 or 2,
   **characterized in that** the
   thermo-hydraulic ageing model (16) is configured as a network model.

4. The method according to any one of the preceding claims,
   **characterised in that**
   in step S3, quantities of 2-FAL and/or $CO_2$+CO are calculated as ageing product quantities.

5. The method according to any one of the preceding claims,
   **characterised in that**
   in step S3, when calculating the ageing product quantities for the different regions of the solid insulation arrangement (I), the equilibrium state of the or of the respective ageing product between the solid insulation arrangement (I) and the insulating medium (13) is taken into account in each case, taking into account the increase of the or of the respective ageing product due to ageing and the mixing of the or the respective aging product in the insulating medium (13) in order to determine the absorption of the at least one ageing product into the insulating medium (13).

6. The method according to any one of the preceding claims,
   **characterised in that**
   in step S3, when calculating the ageing product quantities, a redistribution of the at least one ageing product from locations having a higher generation rate to locations having a lower generation rate via transport through the insulating medium (13) is taken into account.

7. The method according to any one of the preceding claims,
   **characterised in that**
   in step S3, the calculation of the ageing product quantities is carried out using at least one formula which is or has been created based on metrologically acquired data.

8. The method according to any one of the preceding claims,
   **characterised in that**
   in step S3, quantities of 2-FAL are calculated as ageing product quantities and the formula

$$[2FAL(\mu g/g\ paper)]=$$

$$((10^6*((DP_0/DP_t)-1))/162*DP_0)*96*0.3,$$

is used for the calculation, wherein $DP_0$ is the initial DP number, DPt is the DP number at any time during ageing, and "g paper" is the mass of the solid insulation arrangement (I) or of the respective part of the solid insulation arrangement (I), wherein the fixed insulation arrangement (I) comprises paper and/or pressboard or is provided by paper and/or pressboard.

9. The method according to any one of the preceding claims,
**characterised in that**
the insulation medium (13) comprises an oil or is provided by an oil, and/or that the solid insulation arrangement (I) comprises cellulose or is provided by cellulose.

10. The method according to any one of the preceding claims,
**characterised in that**
the electrical device (1) comprises a tank (2) filled with the insulating medium (13), in which components of the electrical device (1) are arranged, wherein one or more of the components are provided with the solid insulation arrangement (I).

11. The method according to any one of the preceding claims,
**characterised in that**
wherein the solid insulation arrangement (I) is configured in multiple parts and, in step S2, the different regions of the solid insulation arrangement (I) comprise different parts of the solid insulation arrangement (I) or are provided thereby, wherein different components of the electrical device (1) are assigned different parts of the solid insulation arrangement (I), wherein different components of the electrical device (1) are provided with different parts of the fixed insulation arrangement (I).

12. The method according to any one of the preceding claims,
**characterised in that**
a simulation of the operation is carried out for a simulation period of several hours, months or years.

13. A computer program comprising program code means which, upon execution of the program on at least one computer, cause the at least one computer to perform the steps of the method according to any one of claims 1 to 12.

14. A computer-readable medium comprising instructions which, when being executed on at least one computer, cause the at least one computer to perform the steps of the method according to any one of claims 1 to 12.

**Revendications**

1. Procédé mis en œuvre par ordinateur pour l'évaluation du vieillissement et la surveillance de l'état d'un appareil électrique (1) comprenant un agencement d'isolation solide (I) et un milieu isolant liquide et/ou gazeux (13) en contact avec l'agencement d'isolation solide (I), l'appareil électrique étant un transformateur ou une bobine d'inductance, dans lequel

S1) un modèle de vieillissement thermo-hydraulique (16) de l'appareil électrique (1) est fourni et/ou créé et une simulation pour l'appareil électrique (1) est réalisée avec le modèle de vieillissement (16),
S2) dans le cadre de la simulation, des températures locales (T1-T5) sont calculées pour différentes zones de l'appareil électrique, pour différentes zones de l'agencement d'isolation solide (I) et/ou pour différentes zones du volume occupé par le milieu isolant (13), dans lequel, en tenant compte des températures locales et d'autres grandeurs d'influence déterminant le vieillissement, à savoir l'humidité locale et/ou la teneur en oxygène du milieu isolant (13), des grandeurs de vieillissement locales (DP1-DP5) sont calculées pour différentes zones de l'agencement d'isolation solide (I),
S3) pour les différentes zones de l'agencement d'isolation solide (I), en tenant compte des températures locales calculées et/ou, si elles ont été calculées à l'étape S2, des grandeurs de vieillissement locales (DP1-DP5) et en tenant compte en outre des masses de l'agencement d'isolation solide (I), des quantités d'au moins un produit de vieillissement, qui se forme en raison du vieillissement de l'agencement d'isolation solide (I) et passe dans le milieu isolant (13), sont calculées, les quantités de produits de vieillissement calculées étant utilisées pour distinguer entre un état normal et un état défectueux de l'appareil électrique (1).

2. Procédé selon la revendication 1,
**caractérisé en ce**
**qu'**une comparaison des quantités de produits de vieillissement calculées à l'étape S3 avec une ou plusieurs quantités de produits de vieillissement détectées par mesure sur l'appareil électrique (1) est effectuée, et une distinction est faite entre un état normal et un état défectueux de l'appareil électrique (1) sur la base du résultat de la comparaison.

3. Procédé selon l'une quelconque des revendications 1 ou 2,
**caractérisé en ce que**
le modèle de vieillissement thermo-hydraulique (16) est conçu comme un modèle de réseau.

4. Procédé selon l'une quelconque des revendications précédentes,
**caractérisé en ce**
**qu'**à l'étape S3, des quantités de 2-FAL et/ou de $CO_2$+CO sont calculées en tant que quantités de produits de vieillissement.

5. Procédé selon l'une quelconque des revendications précédentes,
**caractérisé en ce**
**qu'**à l'étape S3, lors du calcul des quantités de produits de vieillissement pour les différentes zones de l'agencement d'isolation solide (I), l'état d'équilibre du ou de chaque produit de vieillissement respectif entre l'agencement d'isolation solide (I) et le milieu isolant (13) est respectivement pris en compte, en tenant compte de l'augmentation du ou de chaque produit de vieillissement respectif par le vieillissement et du mélange du ou de chaque produit de vieillissement respectif dans le milieu isolant (13), afin de déterminer l'absorption de l'au moins un produit de vieillissement dans le milieu isolant (13).

6. Procédé selon l'une quelconque des revendications précédentes,
**caractérisé en ce**
**qu'**à l'étape S3, lors du calcul des quantités de produits de vieillissement, une redistribution de l'au moins un produit de vieillissement depuis des emplacements présentant un taux de génération plus élevé vers des emplacements présentant un taux de génération plus faible via un transport par le milieu isolant (13) est prise en compte.

7. Procédé selon l'une quelconque des revendications précédentes,
**caractérisé en ce**
**qu'**à l'étape S3, le calcul des quantités de produits de vieillissement est effectué en utilisant au moins une formule qui est ou a été établie sur la base de données détectées par mesure.

8. Procédé selon l'une quelconque des revendications précédentes,
**caractérisé en ce**

   **qu'**à l'étape S3, des quantités de 2-FAL sont calculées en tant que quantités de produits de vieillissement, et la formule

$$[2FAL(\mu g/g\ papier)]=$$

$$((10^6*((DP_0/DP_t)-1))/162*DP_0)96*0,3,$$

   est utilisée pour le calcul, où $DP_0$ est l'indice DP initial, DPt est l'indice DP à un moment quelconque pendant le vieillissement et « g papier » est la masse de l'agencement d'isolation solide (I) ou de la partie respective de l'agencement d'isolation solide (I), l'agencement d'isolation solide (I) comprenant du papier et/ou du carton comprimé ou étant constitué par du papier et/ou du carton comprimé.

9. Procédé selon l'une quelconque des revendications précédentes,
**caractérisé en ce que**
le milieu isolant (13) comprend une huile ou est constitué par une huile, et/ou **en ce que** l'agencement d'isolation solide (I) comprend de la cellulose ou est constitué par de la cellulose.

10. Procédé selon l'une quelconque des revendications précédentes,
    **caractérisé en ce que**
    l'appareil électrique (1) comprend une cuve (2) remplie du milieu isolant (13), dans laquelle sont disposés des composants de l'appareil électrique (1), un ou plusieurs des composants étant munis de l'agencement d'isolation solide (I).

11. Procédé selon l'une quelconque des revendications précédentes,
    **caractérisé en ce que**
    l'agencement d'isolation solide (I) est réalisé en plusieurs parties et, à l'étape S2, les différentes zones de l'agencement d'isolation solide (I) comprennent ou sont constituées par différentes parties de l'agencement d'isolation solide (I), différentes parties de l'agencement d'isolation solide (I) étant associées à différents composants de l'appareil électrique (1), différents composants de l'appareil électrique (1) étant munis de différentes parties de l'agencement d'isolation solide (I).

12. Procédé selon l'une quelconque des revendications précédentes,
    **caractérisé en ce**
    **qu'**une simulation du fonctionnement est effectuée pour une période de simulation de plusieurs heures, mois ou années.

13. Programme informatique comprenant des moyens de code de programme qui, lors de l'exécution du programme sur au moins un ordinateur, amènent l'au moins un ordinateur à réaliser les étapes du procédé selon l'une quelconque des revendications 1 à 12.

14. Support lisible par ordinateur comprenant des instructions qui, lorsqu'elles sont exécutées sur au moins un ordinateur, amènent l'au moins un ordinateur à réaliser les étapes du procédé selon l'une quelconque des revendications 1 à 12.

# FIG 1

$T_{T0}$

$T_H$

$T_{B0}$

FIG 2

## FIG 3

## FIG 4

FIG 5

FIG 6

FIG 7

DP3  DP1  DP5    DPav

DP4  DP2

t [a]

EP 4 428 554 B1

FIG 8

log ($CO_2$ + CO) [a.u.]

## IN DER BESCHREIBUNG AUFGEFÜHRTE DOKUMENTE

**In der Beschreibung aufgeführte Patentdokumente**

- CN 112257232 A
- WO 9960682 A1

**In der Beschreibung aufgeführte Nicht-Patentliteratur**

- Simulation of long-term transformer operation with a dynamic thermal, moisture and aging model. *5th International Colloquium on Transformer Research and Asset Management*, October 2018
- 5th International Colloquium on Transformer Research and Asset Management. Lecture Notes in Electrical Engineering. Springer, 2020, vol. 671
- **NAOKI YAMAGATA et al.** Diagnosis of Thermal Degradation for Thermally Upgraded Paper in Mineral Oil. *International Conference on Condition Monitoring and Diagnosis*, 2008
- **CHRISTOPH KRAUSE**. The Condition of Solid Transformer Insulation at End-of-Life. *CIGRE ELECTRA*, April 2022 (321)